# EUROPÄISCHE PATENTSCHRIFT

(11) **EP 2 645 950 B1**
(45) Veröffentlichungstag und Bekanntmachung des Hinweises auf die Patenterteilung: **03.10.2018**
(21) Anmeldenummer: 11796951.9
(22) Anmeldetag: 01.12.2011
(51) Int. Cl.: A61B 17/70

(54) **FUSIONSIMPLANTAT FÜR FACETTENGELENKE**
FUSION IMPLANT FOR FACET JOINTS
IMPLANT POUR FUSION OSSEUSE DESTINÉ AUX ARTICULATIONS ZYGAPOPHYSAIRES

(30) Priorität: 01.12.2010 EP 10193333
(43) Veröffentlichungstag der Anmeldung: 09.10.2013
(73) Patentinhaber: FACET-LINK Inc., Rockaway NJ 07866 (US)
(72) Erfinder: JENSEN, Harm-Iven, 24214 Noer (DE); LINK, Helmut D., 22397 Hamburg (DE)
(74) Vertreter: Glawe, Delfs, Moll
(86) Internationale Anmeldenummer: PCT/EP2011/071500
(87) Internationale Veröffentlichungsnummer: WO 2012/072733

(56) Entgegenhaltungen:
- WO-A1-2009/094629
- WO-A1-2010/122472
- US-A1- 2005 143 818
- US-A1- 2006 036 324
- US-A1- 2009 012 566

## Beschreibung

Die Erfindung betrifft ein Fusionsimplantat für Facettengelenke, das ein Fusionsmodul zur Verbindung der Facettengelenke benachbarter Wirbel und ein das Fusionsmodul halterndes Tragmodul aufweisen.

Die Wirbelsäule bildet ein zentrales Strukturelement des menschlichen Skeletts. Sie umfasst eine Vielzahl von Wirbeln, die für die Übertragung von Lasten übereinander angeordnet und zur Ermöglichung von Bewegungen gelenkig miteinander verbunden sind. Die Wirbel der Wirbelsäule sind nicht identisch, sondern unterscheiden sich in ihrer Gestalt je nach ihrer Anordnung an der Wirbelsäule. Sie weisen jedoch einige Gemeinsamkeiten auf. So weist jeder Wirbel einen massiven Wirbelkörper mit zwei seitlich nach hinten vorstehenden knöchernen Vorsprüngen (Pedikel) auf, die wiederum in ihrem hinteren Teil über einen knöchernen Bogen verbunden sind. Im Verbindungsbereich ist dieser knöcherne Bogen als breite Platte (Lamina) ausgebildet, und weist in seiner Mitte einen nach hinten abragenden spinalen Vorsprung auf. Der spinale Vorsprung (Prozessus spinalis) wie auch zwei weitere transverse Vorsprünge an den Seitenflächen der Pedikel bilden Anlenkpunkte für Muskeln und Bänder. In dem Bereich, in dem die Pedikel in die breite Lamina übergehen, sind auf jeder Seite des Wirbels ein oberer und ein unterer Gelenkvorsprung angeordnet. Sie bilden jeweils Teil eines Facettengelenks mit einem benachbarten unteren beziehungsweise oberen Wirbel. Weiter ist zur Lastübertragung der Wirbel vorgesehen, dass zwischen den Wirbelkörpern benachbarter Wirbel jeweils eine Bandscheibe angeordnet ist, die den Zwischenraum zwischen den verhältnismäßig ebenen Deckflächen benachbarter Wirbelkörper ausfüllt. Der von den Rückseiten des Wirbelkörpers und dem knöchernen Bogen (Wirbelbogen) umgrenzter Bereich bildet einen Hohlraum, in welchem parallel zur Wirbelsäule laufende Nervenstränge aufgenommen sind.

Aufgrund von Degeneration der Wirbelsäule kommt es häufig zu Rückenbeschwerden. Hierbei liegt eine der Hauptursachen für Rückenschmerzen in der Interaktion zwischen zwei benachbarten Wirbeln. Dies betrifft insbesondere Bandscheiben als eine Hauptursache, aber in einem beträchtlichen Anteil der Fälle umfasst die Pathologie zumindest auch Facettengelenke. Aufgrund von Verschleiß oder Krankheit kann die für die Facettengelenke bewirkte gelenkige Verbindung zweier benachbarter Wirbel beschädigt sein. Dies kann zu Bewegungseinschränkungen, Schmerzen bis hin zu einem Verlust der Mobilität führen. Es sind verschiedene Ansätze zur Therapie bekannt geworden. Insbesondere kann eine deutliche Verbesserung erreicht werden, indem das Facettengelenk stabilisiert wird. In vielen Feldern folgt dies in der Weise, dass das Facettengelenk durch eine feste Verbindung immobilisiert wird. Hierbei spricht man von einer Fusion des Facettengelenks.

Gemäß der WO 2010/122472 A1 ist ein Fusionsimplantat bekannt, das Knochenschrauben umfasst, die an je einem gesonderten Halter angeordnet sind. Die Halter werden über ein Tragmodul mittels einer Befestigungsschraube miteinander verbunden, wobei das Tragmodul an den Wirbelfortsätzen zweier Wirbel befestigt wird. Nachteilig an diesem Fusionsimplantat ist das Erfordernis einer verhältnismäßig starken und intakten Knochenstruktur am Wirbelkörper, insbesondere im Bereich der Befestigungsschrauben.

Aus der WO 2009/094629 A1 ist ein Fusionsimplantat bekannt geworden, das lange Knochenschrauben umfasst, welche durch die beiden ein Facettengelenk bildenden Facetten durchgeschraubt wird. Die Schraube ist als Kompressionsschraube ausgeführt. Sie zieht die miteinander zusammenwirkenden Hälften des Facettengelenks zusammen, so dass das Gelenk immobilisiert ist. Um die dafür erforderlichen Kräfte auf den Wirbelkörper übertragen zu können, ist der Schraubenkopf mit einer schwenkbeweglich angeordneten gesonderten Auflagehülse versehen. Mit ihrer Schwenkbeweglichkeit kann die transfacettale Kompressionsschraube verschiedene Winkelstellungen zu der durch die Auflagehülse bestimmten Auflageebene einnehmen. Man spricht von einer polyaxialen Anordnung der transfacettalen Schraube. Für jedes der beiden Facettengelenke eines Wirbelkörpers (auf einer linken beziehungsweise rechten Seite) ist eine eigene Schraube vorgesehen. Das bekannte Fusionsimplantat bietet den Vorteil verhältnismäßig einfacher Implantierbarkeit, da es nur geringe Abmessungen aufweist und daher auch mittels minimal invasiver Chirurgie implantiert werden kann. Allerdings erfordert dieses bekannte Fusionsimplantat eine verhältnismäßig starke und intakte Knochenstruktur am Wirbelkörper, insbesondere im Bereich der Auflagefläche der schwenkbeweglichen Krause um den Schraubenkopf.

Der Erfindung liegt die Aufgabe zugrunde, ein verbessertes Fusionsimplantat der eingangsgenannten Art zu schaffen, das die oben genannten Nachteile vermeidet.

Die erfindungsgemäße Lösung liegt in den Merkmalen des unabhängigen Anspruchs. Vorteilhafte Weiterbildungen sind Gegenstand der abhängigen Ansprüche.

Bei einem Fusionsimplantat für ein Facettengelenk, umfassend ein Tragmodul und ein Fusionsmodul, wobei das Fusionsmodul an dem Tragmodul angeordnet ist und einen Halter für transfacettale Befestigungsmittel aufweist, ist erfindungsgemäß vorgesehen, dass das Tragmodul ein Expansionselement aufweist und das Fusionselement Backenelemente umfasst, die an voneinander wegweisenden Außenseiten Anlageflächen für die Lamina aufweisen und die längsverschieblich an einer Führung des Expansionselements so angeordnet sind, dass ein Abstand der Backenelemente mittels des Expansionselements veränderlich ist.

Die Erfindung beruht auf dem Gedanken, ein Fusionsimplantat zu schaffen, bei dem die Funktion des Haltens und der Positionierung an dem Wirbelkörper entkoppelt ist von der Funktion des eigentlichen Einwirkens auf die Facettengelenke. Mit dem Tragmodul wird eine sichere und präzise positionierbare Verankerung des Fusionsimplantats an einem Wirbelkörper, insbesondere an Laminaschnittflächen, erreicht. Es wird eine solide und feste Plattform geschaffen, so dass die die Fusion bewirkenden Komponenten sowohl absolut bezogen auf den Wirbelkörper wie auch relativ zueinander präzise und sicher positioniert werden können. Weiter bietet die Modulbauweise den Vorteil, dass sie sich insbesondere gut zur Implantation mittels minimal invasiver Chirurgie eignet.

Außerdem erreicht man mit dem Tragmodul eine Augmentation, so dass das erfindungsgemäße Fusionsimplantat sich insbesondere zur Anwendung an Wirbelkörper mit auch größeren Defekten, insbesondere im Bereich der Lamina, eignet. Die Erfindung bietet damit den großen Vorteil, auch dann angewendet werden zu können, wenn der Wirbelkörper durch vorhergehende Dekompressions-Therapie geschwächt ist. Gerade bei dieser in der Praxis sehr häufig vorkommenden Therapie war eine Facettengelenksfusion bisher kontraindiziert. Die Erfindung ermöglicht dies nun und erhöht somit beträchtlich die Zahl der Patienten, denen geholfen werden kann.

Die Erfindung verknüpft damit auf besondere Weise Vorteile hinsichtlich breiter Verwendbarkeit dank Augmentation auch bei Wirbelkörpern mit beträchtlichen Defekten und einer präziseren Positionierung am Wirbelkörper mit Patientenschonung dank minimal invasiver Implantation. Die Facettenfusion als "Goldstandard" kann so mit anderen Therapietechniken kombiniert werden.

Zuerst seien einige verwendete Begriffe erläutert:
Unter "transfacettal" wird eine Durchquerung des Facettengelenks verstanden. Das Facettengelenk umfasst hierbei eine untere Facette des Wirbels, an dem das erfindungsgemäße Implantat anzuordnen ist, und eine obere Facette des darunter liegenden Wirbels; sie bilden gemeinsam das Facettengelenk.

Unter "cephalad" wird eine Richtung verstanden, die bezogen auf die implantierte Position in Richtung nach oben, also zum Kopf des Patienten weist. Dementsprechend wird unter caudal die entgegengesetzte Richtung verstanden, die also vom Kopf weg nach unten weist.

Das Fusionsmodul weist vorzugsweise ein Hauptlager an einer oberen Facette und ein Gegenlager an einer unteren Facette des zu fusionierenden Facettengelenks auf. Die Einleitung der Fusionskräfte kann damit auf beiden Seiten in definierter und letztlich Knochensubstanz schonender Weise erfolgen. Das Gegenlager kann als gesondertes Element an der jenseitigen Facette angeordnet sein, oder es kann einstückig mit dem die eigentliche Fusion bewirkenden Element ausgebildet sein, beispielsweise als Knochengewinde einer transfacettalen Schraube als Befestigungselement. Die transfacettale Schraube kann kanüliert oder solide ausgeführt sein.

Das Hauptlager ist vorzugsweise schwenkbar. Damit kann die Richtung, in der die Facettenfusion erfolgt, mithin also die funktionale Achse verändert werden. Eine solche polyaxiale Lagerung ermöglicht eine verbesserte Anpassung an die individuellen anatomischen Verhältnisse. Bewährt hat sich ein Verstellbereich von +/- 15 Grad in alle Richtungen. Bei einer zweckmäßigen Ausführung umfasst das Hauptlager einen kugelkalottenförmigen Aufnahmesitz, in dem eine Fixierhülse schwenkbar gehaltert ist. Durch die kugelartige Grenzfläche zwischen Fixierhülse und Aufnahmesitz sind rotatorische Freiheitsgrade in allen Richtungen gegeben. Besonders günstig ist diese Konstruktion, wenn eine transfacettale Schraube zur Fusion vorgesehen ist.

Das Fusionsmodul ist aus Gründen erhöhter Befestigungssicherheit in der Regel starr an dem Tragmodul angeordnet. Es soll aber nicht ausgeschlossen sein, dass es flexibel an dem Tragmodul angeordnet ist. Dies kann Vorzüge hinsichtlich der Kompatibilität mit dynamischen Bewegungen der Wirbelsäule mit sich bringen.

Die Anlagefläche der Backenelemente kann so geformt sein, dass sie im cephaladen Bereich schmaler ist. Damit kann die Stärke des Implantats in einem kritischen Bereich, in dem es ansonsten leicht zu einer unerwünschten Druckausübung auf im Spinalkanal laufende Nervenstränge oder diese umgebendes Gewebe kommen könnte, verringert werden. Die Verträglichkeit des Implantats erhöht sich damit. Mit Vorteil ist das Fusionsmodul im caudalen Bereich der Anlageflächen angeordnet. Hier steht ausreichend Bauraum für das Implantat zur Verfügung, ohne dass eine erhöhte Gefahr von Irritationen von umgebendem Gewebe geschaffen wird.

In vielen Fällen ist bei einem Wirbel, an dem das erfindungsgemäße Fusionsimplantat angeordnet werden soll, die Lamina wegen einer Dekompressionsbehandlung vollständig entfernt. Über das in den Freiraum eingesetzten Tragmodul werden die Resektionsflächen der Lamina miteinander verbunden, so dass funktional der Bogen wieder geschlossen ist. Es kann aber auch sein, dass eine vollständige Resektion der Lamina über ihre volle Höhe nicht erforderlich ist, sondern dass sie über einen Teil ihrer Höhe bestehen bleibt. Dieser Rest alleine ist mechanisch nicht mehr voll belastbar. Hier setzt die Erfindung mit einer Variante des Fusionsimplantats an, die eine geringe Bauhöhe hat. Das zur Einbettung in die Lamina vorgesehene Tragmodul des Fusionsimplantats weist dazu eine geringere Abmessung in cephalad-caudaler Richtung auf, die vorzugsweise weniger als die Hälfte der Erstreckung der Anlagefläche in dieser Richtung beträgt. Damit ist das Fusionsimplantat so kompakt, dass es unter den Laminarest gesetzt werden und ihn verstärken kann, wobei es gleichzeitig eine solide Basis für die Facettenfusion bildet. Vorzugsweise ist das Fusionsmodul im mittleren Bereich der Backenelemente angeordnet, und die Backenelemente sind gegeneinander tauschbar. Damit kann die Relation zwischen Backenelementen und ihrer Führung invertiert werden. Bildet in der Grundversion die Führung mit den Backenelementen eine nach unten offene U- bzw. H-förmige Struktur, so entsteht mit der Inversion dank der Tauschbarkeit der Backenelemente eine nach oben offene U-Form. Diese eignet sich besonders zur Implantation unter einem Resektionsrest der Lamina mit dem spinalen Vorsprung.

Ferner kann zur weiteren Steigerung der Kompaktheit das Fusionsmodul nicht an dem Tragmodul, sondern an mindestens einem der Backenelemente angeordnet sein, und zwar vorzugsweise tragmodulseitig an den Backenelementen. Es sei angemerkt, dass das Fusionsmodul nur einseitig, d. h. an nur einem Backenelement, oder zweiseitig, d. h. an beiden Backenelementen, vorgesehen sein kann.

Das Tragmodul ist vorzugsweise insbesondere mit seiner Führung so auf das Fusionsmodul abgestimmt, dass ein koaxialer Zugangsweg zur transfacettalen Schraube im Fusionsmodul frei ist. Mit anderen Worten, es besteht ein ungehinderter Zugang zum Kopf der transfacettalen Schraube in Richtung der verlängerten Schraubenachse. Damit sind die konstruktiven Voraussetzungen geschaffen, um die transfacettale Schraube im montierten Zustand am Implantationsort festziehen zu können.

Bei einer Ausführungsform ist eine Pedikelstütze an dem Tragmodul angeordnet. Die Pedikelstütze erlaubt eine sichere und robuste Fixierung, stellt aber hohe Ansprüche an die Positionierungsgenauigkeit. Dank der erfindungsgemäßen Anordnung an dem Tragmodul ist eine präzise Positionierung erreicht, so dass das im Stand der Technik vorhandene Risiko einer Fehlpositionierung, die zu beträchtlichen Schädigungen führen kann, eliminiert ist. Das gilt insbesondere dann, wenn an der Pedikelstütze ein Lager für Pedikelschrauben vorgesehen ist. Um dennoch eine ausreichende Anpassmöglichkeit an die individuelle Anatomie des Patienten zu ermöglichen, ist die Pedikelschraube vorzugsweise polyaxial gelagert. Dazu ist das Lager entsprechend dem der transfacettalen Schraube ausgebildet.

Die Pedikelstütze ist mit Vorteil an einer Ausrichteinrichtung des Tragmoduls gehaltert. An dieser Stelle kann die Pedikelstütze modular hinzugefügt oder weggelassen werden. Auch eine einseitige Anordnung der Pedikelstütze ist auf diese Weise leicht möglich.

Bei einer bevorzugten Ausführungsform ist die Pedikelstütze an dem Backenelement angeordnet. Dies ermöglicht eine kompaktere Gestaltung des Tragmoduls, so dass eine Pedikelstütze auch bei beengten Raumverhältnissen angeordnet sein kann. Weiterhin ist der Lastfluss verkürzt, über den die Haltekraft in den das Fusionsimplantat aufnehmenden Wirbelkörper geleitet wird. Es ergibt sich somit nicht nur eine kompaktere, sondern auch eine steifere Konstruktion. Die Befestigung der Pedikelstütze erfolgt vorzugsweise mittels eines Schwenklagers. Damit kann eine Anpassung an die anatomischen Verhältnisse an den jeweiligen Wirbelkörpern durchgeführt werden. Das Fusionsimplantat wird damit nicht nur vielseitiger verwendbar, sondern auch ausgesprochen kompakt. Auch bei beengten anatomischen Verhältnissen kann es angewendet werden.

Das Schwenklager weist zweckmäßigerweise einen geschlitzten kalottenförmigen Lagerkörper auf. Damit kann in einer kugelförmigen Aufnahme, die augenförmig an einem Ende der Pedikelstütze ausgeführt sein kann, eine zuverlässige und kompakte Lagerung gebildet sein. Die Schlitzung kombiniert gute Montier- und Verstellbarkeit mit sicherer Fixierung. Der kalottenförmige Lagerkörper ist mit einer Durchgangsöffnung für eine Befestigungsschraube versehen, wobei die Durchgangsöffnung an einem Rand einen umlaufenden Ringkragen aufweist. Damit ist eine sichere Positionierung des Lagerkörpers in seiner Normallage ermöglicht. Weiterhin kann die Durchgangsöffnung mit einer nach innen weisende Radialleiste mit einem vorzugsweise kurzen Gegengewinde versehen sein. Unter "kurz" werden bis zu zwei Umdrehungen verstanden. Damit kann eine stärkere Fixations- und Klemmwirkung erzielt werden, so dass die Pedikelstütze ihre Lage noch sicherer beibehält.

Vorzugsweise weist die Aufnahme in der Pedikelstütze mehrere Abschnitte auf, von denen einer als konische Durchgangsbohrung und der andere als Gewindeabschnitt ausgebildet ist.

Bei einer zweckmäßigen Ausführungsform ist die Pedikelstütze so ausgeführt, dass die Pedikelschrauben parallele Achsen aufweisen, die etwa quer zur Verstellrichtung der Führung liegt. Dies ermöglicht eine kompakte Ausführung der Pedikelstütze. Vorzugsweise weist sie eine solche Abmessung auf, dass ihre lateralen Abmessungen nicht mehr als das 1,5-fache der lateralen Abmessung des Tragmoduls ist. Alternativ kann die Pedikelstütze auch lateral ausgreifend vorgesehen sein, wobei sie deutlich weiter nach lateral überragt. Hierbei sind die Achsen der Pedikelschraube zur Mitte des Tragmoduls hin konvergierend ausgerichtet. Dies ermöglicht eine besonders stabile Befestigung.

Die Erfindung erreicht Befestigungssicherheit durch elastische Aufweitung der Lamina bzw. des Wirbelbogens. Dazu weist das Tragmodul ein Expansionselement auf, welches vorzugsweise eine Führung gebildet aus einem in einer Nut längsbeweglichen Stab umfasst. Mit dieser Führung kann das Expansionselement und damit die Backenelemente so weit auseinandergespreizt werden, dass der resezierte Bereich der Lamina überbrückt werden kann. Vorzugsweise sind Stab und Nut mittels einer formschlüssig eingreifenden Leiste verdrehgesichert zueinander. Um eine Überexpansion zu vermeiden, ist zweckmäßigerweise der Stab an seinem freien Ende mit einer Verdickung versehen. Diese kann nicht in in die Nut eintreten und verhindert so ein Herausrutschen durch Überexpansion. Die Verdickung kann einteilig mit dem Stab ausgeführt sein, ist vorzugsweise aber als eine in die Stirnseite am freien Ende einzusetzende Schraube ausgeführt.

Das Expansionselement hat in Kombination mit den außen gelegenen Anlageflächen der Backenelemente den Vorteil, dass ein Kollabieren des Wirbelbogens, wie es bisher in unerwünschter Weise geschehen konnte, unmöglich gemacht ist. Im Gegenteil, unter dem bisher zum Kollabieren führenden Druck wird das Verstärkungsimplantat nur fester in seinen Sitz gepresst und kann damit seine Aufgabe erfüllen. Obgleich die elastische Aufweitung einen langzeitstabilen Sitz an sich gewährleisten kann, kann zusätzlich eine Rücklaufsperre für die Backenelemente vorgesehen sein, um so die langfristige Befestigungssicherheit weiter zu erhöhen.

Die Rücklaufsperre ist mit Vorteil als eine zwischen den Backenelementen und der Führung wirkende Ausrichteinrichtung ausgebildet. Mit einer solchen Klemmung lässt sich nach dem Expandieren auf einfache Weise die erreichte Expansionsposition fixieren. Damit wird ein Verrutschen der Backenelemente verhindert. Sollten die Anforderungen an die Sicherheit gegenüber unerwünschten Bewegungen der Backenelemente höher sein, so kann vorzugsweise die Rücklaufsperre mit Verrastungselementen versehen sein, die zwischen den Backenelementen und der Führung angeordnet sind. Zweckmäßigerweise umfassen die Verrastungsmittel eine Riffelung und in sie eingreifende Rastnasen. Mit einer solchen Verrastung, wie sie durch die Riffelung in Kombination mit der Rastnase erreicht ist, ergibt sich eine formschlüssige Sicherung. Dies bietet den Vorteil, auch bei sehr aktiven Patienten mit entsprechender Belastung der Wirbelsäule eine ausreichend sichere Halterung des Verstärkungsimplantats zu erreichen.

Vorzugsweise ist mindestens eines der Backenelemente mit einer Ausrichteinrichtung versehen. Damit kann die Orientierung von dessen Außenfläche zu der Führung verändert werden. Dies ermöglicht eine feinere Anpassung des Fusionsimplantats an die tatsächlichen anatomischen Verhältnisse nach der Laminektomie.

Besonders bewährt hat sich eine Ausführung eines Lagers der Ausrichteinrichtung als Klemmschraube. Im ungespannten Zustand ermöglicht sie eine Verdrehung um ihre Mittelachse, während sie im gespannten Zustand die erreichte Orientierung sichert. Zweckmäßigerweise ist das andere Backenelement entsprechend ausgerüstet.

Es hat sich bewährt, die Ausrichteinrichtung mit einer Verdrehsicherung zu versehen. Sie ist dazu ausgebildet, den Winkel zu begrenzen, mit dem die Backenelemente relativ zum Expansionselement einnehmen können. Bewährt hat sich eine Begrenzung auf einen Verstellbereich von maximal 45 Grad, vorzugsweise maximal 30 Grad.

Die Anlageflächen an den Backenelementen weisen vorzugsweise spitze Hervorstehungen (Spikes) auf. Bewährte Formen für solche Spikes sind bspw. Kegelspitzen, Pyramiden, prismatische oder V-förmige Erhebungen. Damit kann eine sichere primäre Fixation erreicht werden. Um zusätzlich auch eine schnelle und sichere sekundäre Fixation zu erreichen, sind vorzugsweise die Anlageflächen mit einer knochenwachstumsfördernden Beschichtung versehen. Hierbei kann es sich insbesondere um Hydroxylapatit oder andere osteoinduktive Substanzen handeln.

Die Anlageflächen sind vorzugsweise so an den beiden Backenelementen angeordnet, dass sie miteinander fluchten. Hierunter wird verstanden, dass sie in Richtung des Verstellwegs des Expansionselements gesehen weder einen horizontalen noch vertikalen Offset aufweisen. Damit wird eine asymmetrische Kraftbeaufschlagung des Fusionsimplantats verhindert, so dass keine unerwünschten Drehmomente auf das Fusionsimplantat wirken, welche es aus seiner vorgesehenen Position zu drehen trachten.

Gegenstand der Erfindung ist ferner ein Satz umfassend ein Implantat wie vorstehend beschrieben und ein Instrumentarium, welches umfasst einen langgestreckten Führungsdraht, einen Führungsschaft, durch welchen der Führungsdraht steckbar ist, mit einem zur Aufnahme am Hauptlager des Fusionsmoduls ausgebildetem Ende, und einen kanülierten Schraubendreher. Nach dem teilweisen oder vollständigen Resezieren der Lamina wird das Implantat in den so geschaffenen Freiraum eingesetzt. Hierzu kann eine optionale Setzpinzette vorgesehen sein. Im nächsten Schritt wird mittels einer optionalen Spreizzange, die bei gesonderter Ausführung dank ihrer Abwinkelung gleichzeitig mit der Setzpinzette angesetzt sein kann, das Expansionselement des Implantats gespreizt. Es sei angemerkt, dass die Spreizzange zusammen mit der Setzpinzette als kombiniertes Instrument ausgeführt sein kann. Es wird dann der Führungsdraht durch den Führungsschaft geschoben, und zwar durch das Hauptlager zuerst in die obere Facette und weiter in die untere Facette. Damit wird der Implantationspfad für die transfacettale Schraube definiert. Danach wird der Führungsschaft entfernt und ein Gewebeschutzrohr aufgeschoben. Sein Innendurchmesser ist so groß bemessen, dass die transfacettale Schraube hindurch geführt werden kann. Die transfacettale Schraube ist kanüliert, das heißt sie weist eine Durchgangsöffnung längs ihrer Mittelachse auf, und kann so auf den Führungsdraht aufgeschoben und auf diesem durch das Gewebeschutzrohr an das Fusionsmodul geführt werden. Mittels eines ebenfalls kanülierten Schraubendrehers, der auf den Führungsdraht in der gleichen Weise aufgesteckt ist, wird die transfacettale Schraube festgeschraubt. Hierbei bleibt dank des Führungsdrahts die Position kontrolliert. Ist die Schraube festgezogen, ist die Position fixiert und der Führungsdraht mit dem Gewebeschutzrohr kann entfernt werden. Nachdem die transfacettale Schraube auf der anderen Seite auf dieselbe Weise befestigt ist, kann die Spreizzange entfernt werden. Es sei angemerkt, dass auch solide transfacettale Schrauben eingesetzt werden können, die dann aber beim Einsetzen nicht durch den Führungsdraht geführt sind. Abschließend können die Klemmschrauben festgezogen werden und das Expansionselement des Fusionsimplants damit festgesetzt werden. Das Implantat ist damit eingesetzt.

Die Erfindung lässt sich für ein Verfahren zur Implantation einsetzen, das mit den vorgenannten Schritten durchgeführt wird. Es eignet sich insbesondere zur Implantation mit einem wenig invasiven dorsalen Zugang ("limited invasive dorsal approach") .

Die Erfindung wird nachfolgend in Bezugnahme auf die beigefügten Zeichnungen anhand von vorteilhaften Ausführungsbeispielen erläutert. Es zeigen:
- Fig. 1 a, b: eine perspektivische Ansicht eines ersten Ausführungsbeispiels in demontiertem und montiertem Zustand;
- Fig. 2 a, b: eine Seitenansicht sowie eine Aufsicht des Ausführungsbeispiels gemäß Fig. 1;
- Fig. 3: eine Detailvergrößerung zu dem in Fig. 1 dargestellten Ausführungsbeispiel;
- Fig. 4 a, b: eine perspektivische Ansicht eines zweiten Ausführungsbeispiels in demontiertem und montiertem Zustand;
- Fig. 5: eine Aufsicht auf das in Fig. 4 dargestellte zweite Ausführungsbeispiel;
- Fig. 6: eine Detailvergrößerung der zu dem in Fig. 4 dargestellten zweiten Ausführungsbeispiel;
- Fig. 7: eine Variante des zweiten Ausführungsbeispiels;
- Fig. 8: eine Explosionsansicht eines dritten Ausführungsbeispiels;
- Fig. 9: eine perspektivische Ansicht des in Fig. 8 dargestellten vierten Ausführungsbeispiels im montierten Zustand;
- Fig. 10: eine Explosionsansicht eines vierten Ausführungsbeispiels;
- Fig. 11 a, b: Detailansichten zu Befestigungsschrauben des in Fig. 10 dargestellten vierten Ausführungsbeispiels;
- Fig. 12 a, b: Varianten zu dem in Fig. 10 dargestellten vierten Ausführungsbeispiel;
- Fig. 13: eine Explosionsansicht zu Befestigungsschrauben für das in Fig. 8 dargestellte dritte Ausführungsbeispiel;
- Fig. 14 a,: b eine Montage- sowie eine Detailansicht mit den Befestigungsschrauben gemäß Fig. 13;
- Fig. 15: ein Instrumentarium zur Implantation in Implantaten gemäß den Ausführungsbeispielen;
- Fig. 16: ein Verwendungsbeispiel für Instrumente aus dem Instrumentarium;
- Fig. 17 a - d: Darstellungen zu Arbeitsschritten bei der Implantation; und
- Fig. 18 a - c: Darstellungen von Implantaten gemäß den Ausführungsbeispielen in implantiertem Zustand.

Ein erstes Ausführungsbeispiel für ein erfindungsgemäßes Fusionsimplantat umfasst in seiner Gesamtheit mit der Bezugsziffer 1 bezeichnetes Tragmodul und ein Fusionsmodul 6. Das Tragmodul 1 ist in Stegbauweise ausgeführt. Es umfasst einen Kulissenkörper 2, in dem ein Schlitten 3 längsverschieblich geführt ist. An dem Kulissenkörper 2 sowie an dem Schlitten 3 ist jeweils ein Backenelement 4 angeordnet. Sie weisen an ihrem jeweils nach außen, d.h. voneinander wegweisenden Seiten eine Anlagefläche 43 an die Lamina eines Wirbels auf.

Der Kulissenkörper 2 und der Schlitten 3 wirken derart zusammen, dass sich bei der Längsverschiebung der Abstand zwischen den Backenelementen 4 ändert. Der Kulissenkörper 2 und der Schlitten 3 wirken daher nach Art eines Expansionselements zusammen, welches den Abstand zwischen den beiden Außenflächen 43 der beiden Backenelemente 4 verschieden groß einstellen kann. Je nach Einstellung des Expansionselements, d.h. relativer Positionierung zwischen dem Kulissenkörper 2 und dem längsverschieblich daran geführten Schlitten 3 kann ein Tragmodul 1 geschaffen werden, welches unterschiedlich breite, durch Resektion entstandene Zwischenräume in der Lamina 93 eines Wirbelkörpers überbrücken kann.

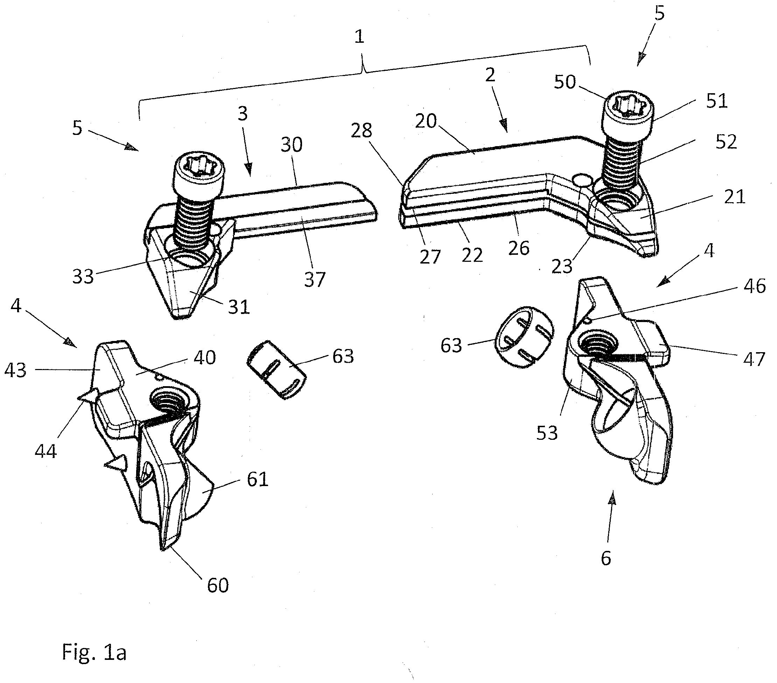

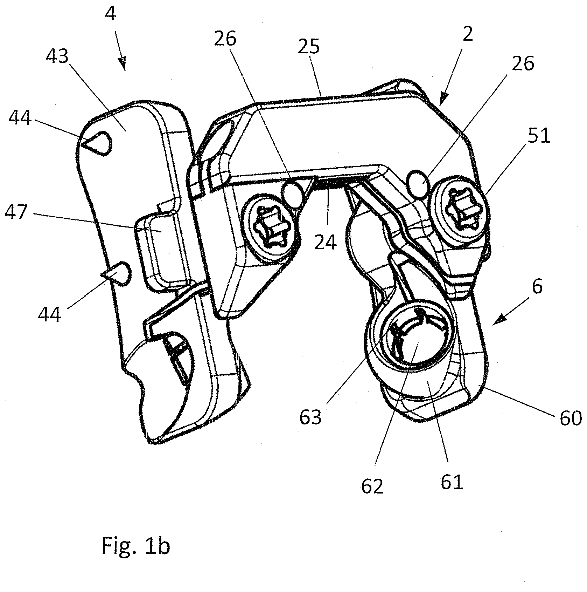

Der Kulissenkörper 2 weist einen Tragsteg 20 auf, an dessen einem (in Fig. 1 rechten) Ende ein Halter 21 ausgebildet ist. Der Steg 2 ist im Querschnitt rechteckförmig und weist zwei Lateralseiten 24, 25 auf. Parallel zu den Lateralseiten 24, 25 ist in dem Steg 20 eine bei dem dargestellten Ausführungsbeispiel kreisförmig ausgebildete Führungsnut 28 angeordnet. Sie ist über einen Schlitz 27 mit der Lateralseite 24 des Stegs 20 verbunden, wobei sich der Schlitz 27 bis in den Halter 21 erstreckt. Der Übergangsbereich zwischen der Lateralseite 25 des Stegs 20 und dem Halter 21 an besagtem Ende des Stegs 20 kann mit einer Abschrägung versehen sein.

Der Schlitten 3 weist als Hauptkomponenten einen Führungsstab 30 und einen Halter 31 auf, der hinsichtlich seiner Außenform spiegelsymmetrisch zu dem Halter 21 des Kulissenkörpers 2 ausgeführt ist. Der Führungsstab 30 weist eine zu der Nut 28 des Kulissenkörpers 2 komplementäre Form auf, so dass eine längsverschiebliche Führung gebildet ist. In dargestelltem Ausführungsbeispiel ist wegen der kreisrunden Form der Führungsnut 28 der Führungsstab 30 mit einem kreisförmigen Querschnitt versehen. Zur Erzielung einer Verdrehsicherheit ist an der zu dem Halter 31 gewandten Seite des Führungsstabs 30 eine schmale Führungsleiste 37 ausgebildet. Ihre Abmessungen sind so gewählt, dass sie schmaler ist als die Weite des Schlitzes 27 am Kulissenkörper 2 in einem entspannten Zustand, aber mindestens so groß wie die Weite in einem gespannten Zustand, wie später noch näher erläutert werden wird. Damit ist der Schlitten 3 längsverschieblich, aber rotationsgesichert an dem Kulissenkörper 2 geführt.

Die Halter 21, 31 an dem Kulissenkörper 2 beziehungsweise dem Schlitten 3 sind im Wesentlichen spiegelsymmetrisch aufgebaut. Sie werden daher nachfolgend gemeinsam erläutert. Die Halter 21, 31 umfassen jeweils eine Durchgangsöffnung 23 beziehungsweise 33, deren Achse lotrecht zu der Achse der längsverschieblichen Bewegung zwischen dem Kulissenkörper 2 und dem Schlitten 3 orientiert ist. Die Durchgangsöffnungen 23, 33 dienen einer Ausrichteinrichtung 5 zum Positionieren und Sichern jeweils eines der Backenelemente 4.

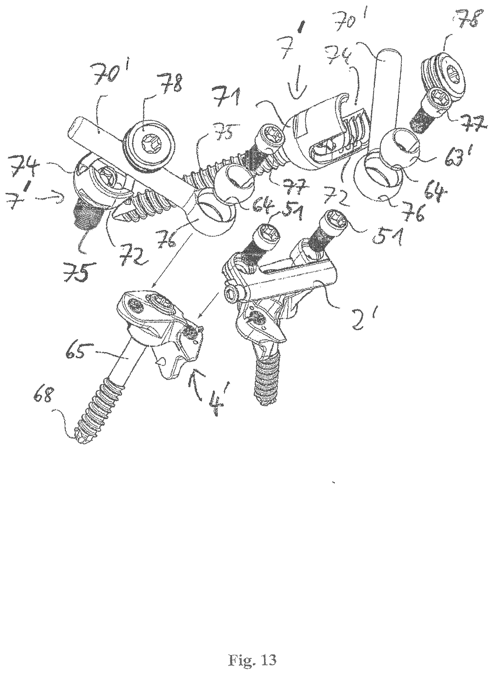

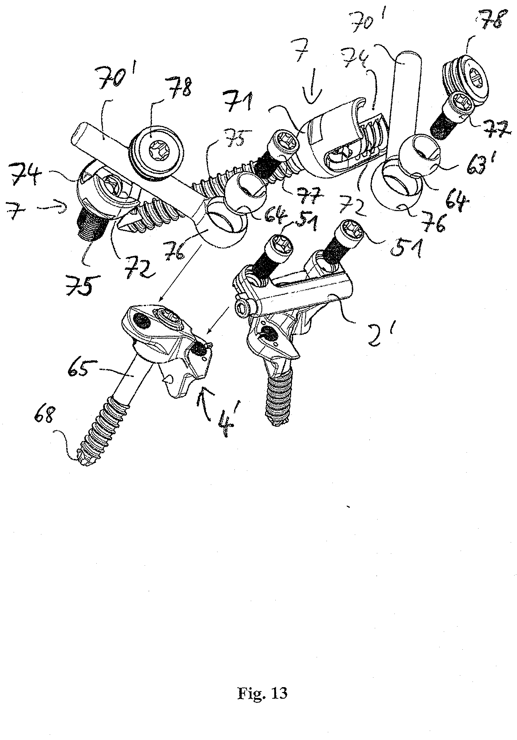

Die Backenelemente 4 sind ebenfalls spiegelsymmetrisch zueinander aufgebaut. Sie weisen an ihrer jeweils von dem anderen Backenelement 4 wegweisenden Anlagefläche 43 eine Mehrzahl von Spikes 44 zur Verankerung in lateralen Schnittflächen 94 der Lamina 93 des Wirbels 9 auf (siehe Fig. 13). Die Anlageflächen 43 mit den Spikes 44 sind vorzugsweise mit einer knochenwachstumsfördernden Beschichtung, wie Calciumphosphat oder Hydroxylapatit versehen. Am Übergang der Anlageflächen 43 zur Oberseite der Backenelemente 4 ist jeweils eine nach außen ragende Nase 47 angeordnet. Sie fungiert als Tiefenanschlag und begrenzt die Einschubtiefe der Backenelemente 4 und damit des gesamten Implantats an der Lamina 93. Ein versehentliches zu weites Einschieben, welches zu Irritationen oder Verletzungen von im Markkanal befindlichen Gewebe- oder Nervensträngen führen könnte, wird damit vermieden. Weiter ist an der Oberseite der Backenelemente 4 jeweils ein Rastvorsprung 46 angeordnet. Er ist dazu ausgebildet, mit einer an der Unterseite 22 des Stegs 20 angeordneten Riffelung 26 zusammenzuwirken. Damit soll erreicht werden, dass im montierten Zustand die Backenelemente 4 gegenüber einem unbeabsichtigten Verdrehen sowie Verschiebung im Fall des Backenelements 4' gegenüber dem Steg 20 gesichert sind. Der mit der Riffelung 26 zusammenwirkende Rastvorsprung 46 bildet eine Verrastung, welche die Position der Backenelemente 4 formschlüssig sichert, so dass eine unbeabsichtigte Verstellung auch bei hoher Belastung ausgeschlossen ist.

Zum Arretieren der Position der Backenelemente 4 gegenüber dem Steg 20 sind Ausrichteinrichtungen 5 an den Haltern 21, 31 vorgesehen. Sie weisen jeweils eine in ihrer Gesamtheit mit der Bezugsziffer 50 bezeichnete Klemmschraube auf, welche einen Schraubenkopf 51 und einen Schaft 52 mit einem Außengewinde aufweist. Der Kopf 51 weist einen größeren Durchmesser als die Durchgangsöffnung 23, 33 in dem Halter 21 beziehungsweise 31 auf, so dass der Schaft 52 durch sie hindurch in ein entsprechendes Gegengewinde in dem Backenelement 4 greifen kann. Durch Festziehen der Klemmschraube 50 wird somit das Backenelement 4 gegen die Unterseite des aus dem Kulissenkörper 2 und dem Schlitten 3 gebildeten Expansionselements gezogen, wodurch die expandierte Position der Backenelemente sowohl hinsichtlich ihres relativen Abstandes voneinander wie auch in der relativen Positionierung (auch in Bezug auf den Steg 20, sowohl kraft- wie auch formschlüssig fixiert ist).

Unter der Wirkung der Ausrichteinrichtung 5 ist ebenfalls der Schlitz 27 des Kulissenkörpers 2 zusammengedrückt, und zwar soweit, dass er die Führungsleiste 37 einklemmt. Damit wird eine weitere Sicherung des Schlittens 3 gegenüber einer unerwünschten Längsverschiebung relativ zu dem Kulissenkörper 2 erreicht.

Die Anlagefläche 43 der Backenelemente 4 sind im Wesentlichen in eine Richtung parallel zu den Außenflächen der Halter 21, 31 länglich ausgestreckt. Dennoch ist die Tiefe der Elemente 4 an einem cephaladen, stegnäheren Ende dünner ausgeführt als an dem gegenüberliegenden caudalen, stegfernen Ende. An diesem ist ein Fusionsmodul 6 angeordnet, welches nachfolgend näher beschrieben wird.

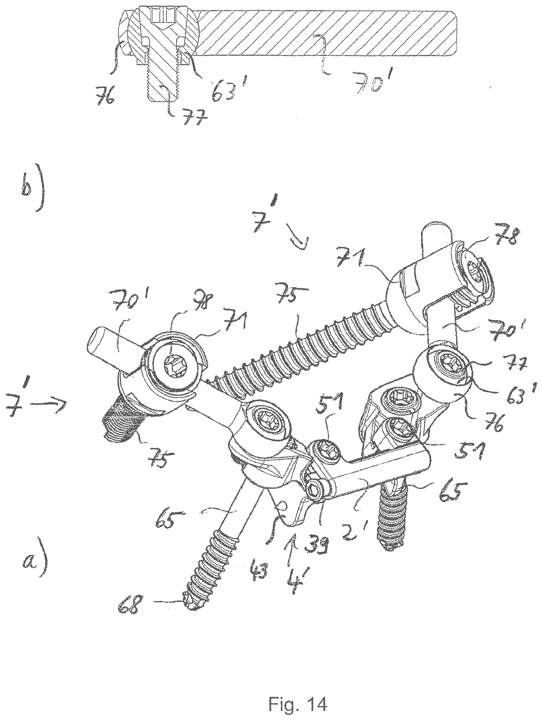

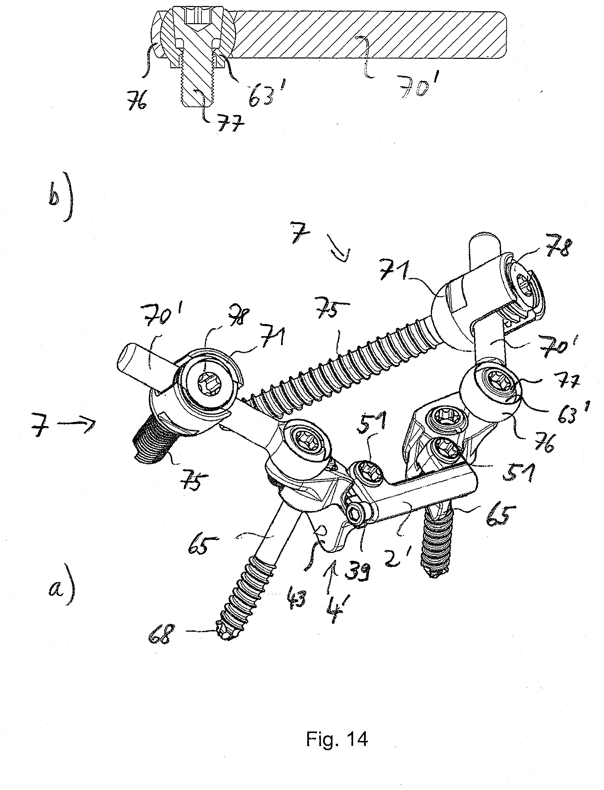

Das Fusionsmodul 6 dient dazu, das untere Facettengelenk des Wirbels 9, an dem das Implantat angeordnet ist, mit den oberen Facetten des darunter angeordneten Wirbels 9' zu fusionieren (siehe insbesondere Fig. 14). Dazu weist das Fusionsmodul 6 ein Hauptlager an einer oberen Facette 96 und ein Gegenlager an einer unteren Facette 97 des Facettengelenks 98 auf. Das Hauptlager 60 ist an dem stegfernen Ende der Backenelemente 4 angeordnet. Es umfasst einen Aufnahmesitz 61, der eine Öffnung 62 mit einem kugelkalottenförmig ausgebildeten Innenraum aufweist. Darin ist eine Fixierhülse 63 angeordnet, deren Außenmantel kugelförmig geformt ist. Sie ist damit schwenkbar in dem Aufnahmesitz 61 gehaltert. Durch die Fixierhülse 63 ist eine transfacettale Schraube 65 gesteckt, die einen Kopf 66 an ihrem proximalen Ende und ein Gewinde 67 in ihrem distalen Bereich aufweist. Die Länge der Schraube 65 ist so gewählt, dass sie etwa der Dicke der beiden Facetten 96, 97 entspricht, die gemeinsam das Facettengelenk 98 bilden. Die Schraube 65 weist längs ihrer Mittelachse eine Durchgangsöffnung 68 auf ("kanülierte Schraube"). Die schwenkbewegliche Lagerung der Fixierhülse 63 in dem Aufnahmesitz 61 ermöglicht es, dass die Schraube 65 in verschiedener Achsorientierung gehaltert sein kann. Im dargestellten Ausführungsbeispiel kann die Schraube bezogen auf die Mittelachse der Öffnung 62 um einen Winkel von ± 15° in alle Richtungen verschwenkt werden. Bei erhöhter Baugröße sind auch größere Verschwenkwinkel möglich, insbesondere bis zu 20° oder 25°. Die transfacettale Schraube 65 dient dazu, nach dem Einsetzen des Tragmoduls 1 eingeschraubt zu werden und mit ihren in die Facette 97 des benachbarten Wirbelkörpers 9' eingreifenden Gewinde 67 ein Gegenlager zu dem an dem Fusionsmodul 6 gebildeten Hauptlager zu bilden, um so beim Einziehen der Schraube die Facetten 96, 97 fest gegeneinander zu ziehen und das Facettengelenk zu immobilisieren. Die transfacettale Schraube 65 kann an ihrem Schaft und/oder Gewinde 67 eine knochenwachstumsfördernde Beschichtung aufweisen.

Die Backenelemente 4 sind um die Achse der Schrauben 50 schwenkbeweglich an den Haltern 21, 31 gehaltert. Um den Winkelbereich auf ein praktikables Maß zu begrenzen, um so insbesondere eine unerwünschte Verdrehung der Backenelemente 4 gerade beim Einsetzen des Tragmoduls 1 an seinen vorgesehenen Implantationsort zu wirken, ist vorzugsweise eine Winkelbegrenzungseinrichtung vorgesehen. Sie ist bei dem dargestellten Ausführungsbeispiel gebildet von einem drehfest an dem Backenelement 4 angeordneten Radialvorsprung 45, der zwischen Seitenwänden einer Ausnehmung 55 an der Unterseite der Halter 21, 31 geführt ist. Hierbei wirken die Seitenwände der Ausnehmung 55 als Anschläge, welche den Schwenkwinkel der Backenelemente 4 relativ zu den Haltern 21, 31 begrenzt sind. In dem dargestellten Ausführungsbeispiel sind die Abmessungen der Ausnehmung 55 so gewählt, dass sich ein Schwenkwinkel von insgesamt 30° für jedes der Backenelemente 4 ergibt.

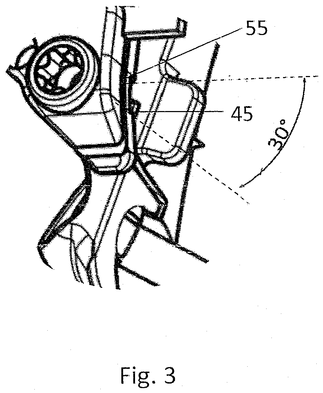

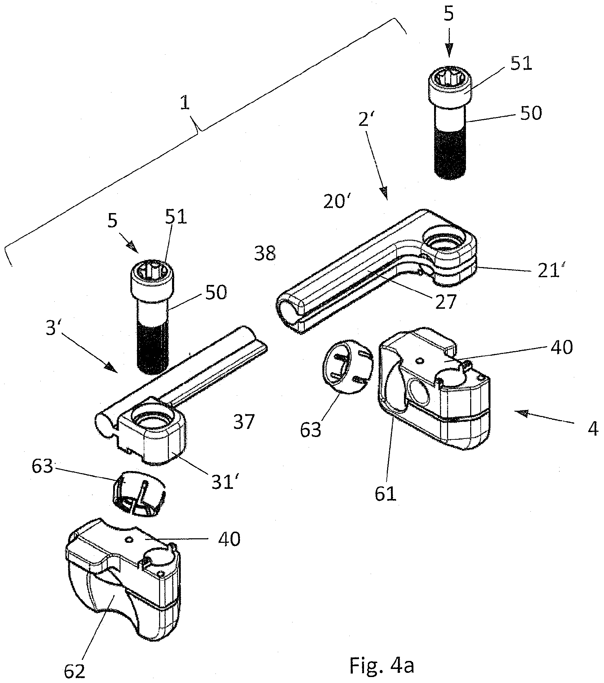

In Fig. 4a, b ist ein zweites Ausführungsbeispiel dargestellt. Dieses unterscheidet vom ersten Ausführungsbeispiel gemäß den Figuren 1 bis 3 im Wesentlichen dadurch, dass das Tragmodul eine deutlich geringere Bauhöhe aufweist. Die Halter 21', 31' sind kompakter ausgeführt als die Halter 21, 31 bei dem ersten Ausführungsbeispiel. Weiter ist der Tragsteg 20' im Querschnitt nicht rechteckförmig, sondern quadratisch ausgeführt. Damit erfordert das aus dem Kulissenkörper 2' in dem Schlitten 3' gebildete Tragmodul 1' eine niedrigere Bauhöhe im Vergleich zu dem Tragmodul 1 des ersten Ausführungsbeispiels. Dies ermöglicht es, das Implantat in eine Teilresektion der Lamina zu implantieren. Dadurch kann eine Laminabrücke bestehen bleiben, so dass zum einen die natürliche Stabilität des Wirbelkörpers weitgehend erhalten bleibt und zum anderen der nach hinten abragende spinale Vorsprung an dem Wirbel erhalten bleiben kann. Die Implantation ist weniger invasiv und schonender für den Patienten. Bei diesem zweiten Ausführungsbeispiel ist, wie aus Fig. 4 ersichtlich, das Fusionsmodul 6 nicht am Rand der Backenelemente 4 angeordnet, sondern vielmehr etwa in deren Mitte. Dies ermöglicht eine kleinere Ausführung der Backenelemente 4. Im Übrigen ist das zweite Ausführungsbeispiel ähnlich zu dem ersten Ausführungsbeispiel ausgeführt, so dass auf vorstehende Erläuterungen verwiesen werden kann.

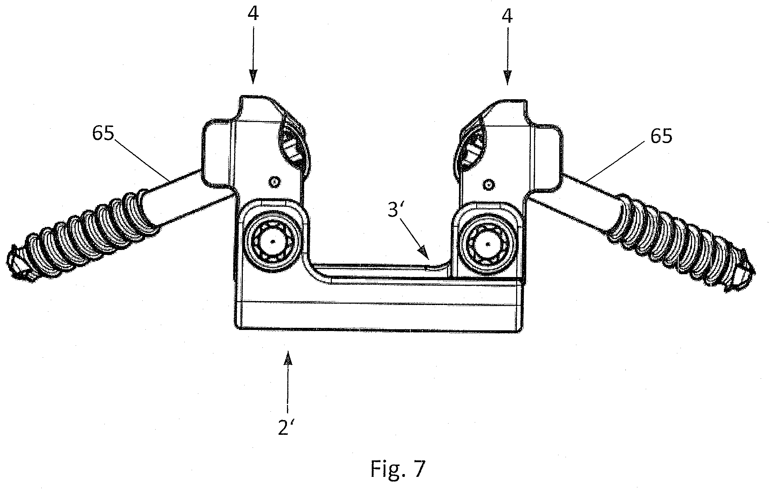

Eine Variante des zweiten Ausführungsbeispiels ist in Fig. 7 dargestellt. Hierbei sind der Kulissenkörper 2' und der Schlitten 3' mit inverser Lage an den Backenelementen 4 angeordnet. Das Tragmodul 1' bildet daher eine deutliche U-Form, wobei das U in Bezug auf die Implantationslage nach oben offen ist. Bei der Grundform, wie in Fig. 4a abgebildet, liegt eher eine H-Form bzw. eine nach unten offene U-Form vor.

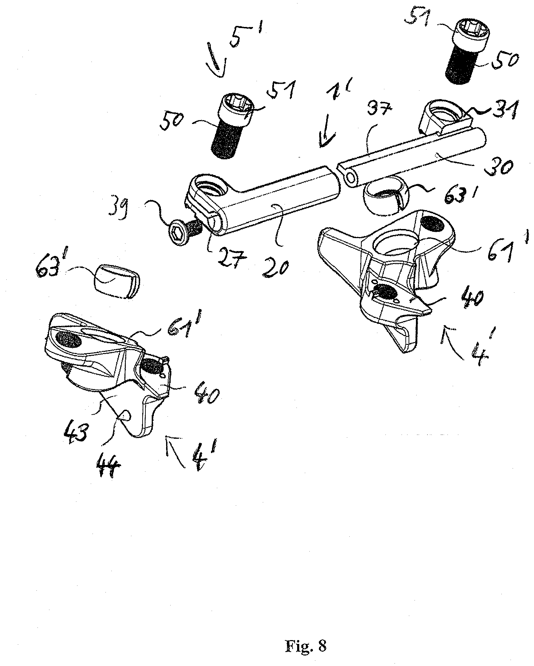

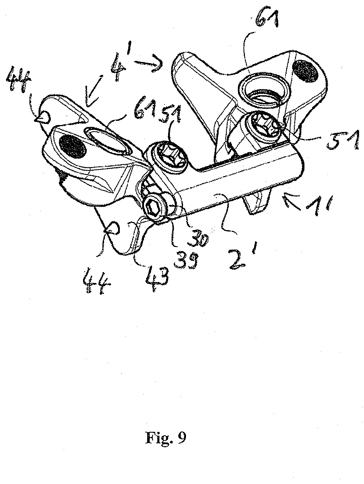

In Fig. 8 und 9 ist ein drittes Ausführungsbeispiel dargestellt. Es basiert auf der in Fig. 7 dargestellten Variante und ist je nach Dimensionierung geeignet sowohl für den Fall einer Teilresektion der Lamina oder wie auch für den Fall einer Resektion der Lamina über die volle Höhe. Gleichartige Elemente mit denen des zweiten Ausführungsbeispiels sind mit gleichnamigen Bezugszeichen bezeichnet. Wie bei der in Fig. 7 dargestellten Variante ist das Tragmodul 1' invers angeordnet, d. h. es bildet ein in Implantationsrichtung nach oben offenes U. Der Stab 30 ist an seinem freien Ende mit einem stirnseitigen Gewinde versehen, in welches eine als Verdickung 39 wirkende Schraube eingesetzt ist. Ihr Kopf weist einen so großen Durchmesser auf, um einen Durchgang durch den Schlitz 27 zu verhindern. Die über eine Ausrichteinrichtung 5' gehalterten Backenelemente 4' sind mit einem Aufnahmesitz 61' für ein Fusionsmodul versehen, wie weiter unten noch näher beschrieben wird, während bei dem zweiten Ausführungsbeispiel eine Anordnung am Tragmodul 1 selbst gewählt ist.

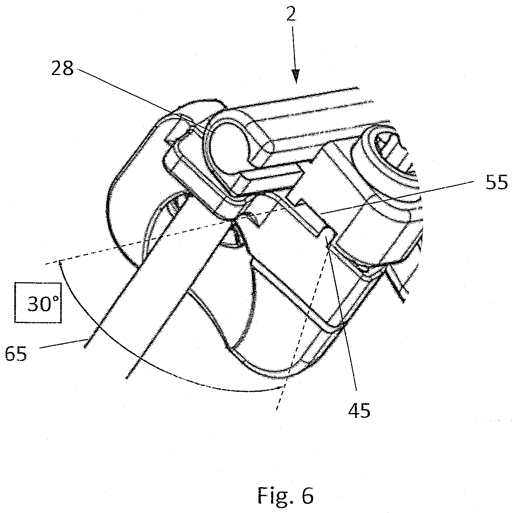

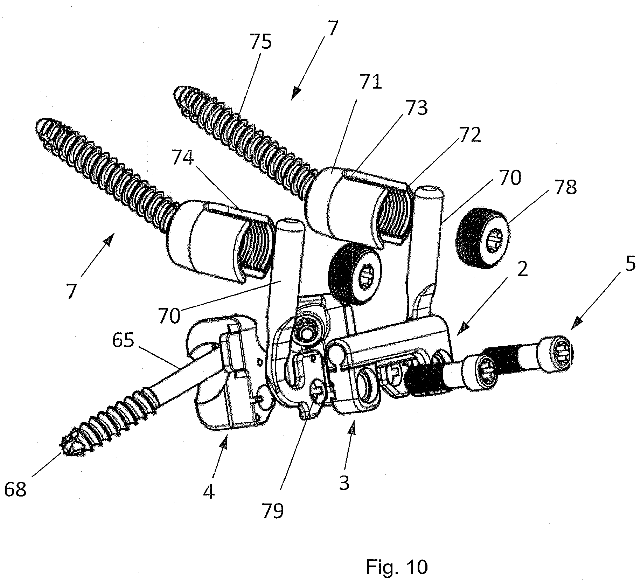

Ein viertes Ausführungsbeispiel ist in Fig. 10 dargestellt. Es basiert auf dem in Fig. 4 bis 6 dargestellten zweiten Ausführungsbeispiel und unterscheidet sich von diesem im Wesentlichen dadurch, dass eine zusätzliche Pedikelstütze 7 vorgesehen ist. Die Pedikelstütze 7 stellt eine zusätzliche Befestigungsmöglichkeit des Implantats an dem Wirbelkörper dar. Sie erhöht die Stabilität. Die Pedikelstütze 7 umfasst jeweils eine Pedikelschraube 75 für den linken und den rechten Pedikel 91 des Wirbelkörpers 9. Nachstehend wird der Aufbau in Bezug auf die rechte Pedikelschraube 75 erläutert; für die linke Pedikelschraube 75 gilt entsprechendes. Die Pedikelschraubenstütze 7 ist über einen Tragstab 70 an dem Tragmodul 1 angeordnet. Der Tragstab 70 weist an seinem unteren Ende einen plattenförmigen Fortsatz auf, in dem eine Durchgangsöffnung 79 ausgebildet ist. Der plattenförmige Fortsatz liegt mit seiner Unterseite auf der Oberseite des Backenelements 4 an und mit seiner Oberseite liegt er an der Unterseite des Kulissenkörpers 2 an. Die Schraube 50 in der Ausrichteinrichtung 5 ist durch die Durchgangsöffnung 79 in dem Fortsatz geführt. Damit wird beim Festziehen des Backenelements 4 an dem Kulissenkörper 2 auch der Tragstab 70 befestigt. An seinem gegenüberliegenden Ende ist der Tragstab 70 kreisförmig ausgebildet.

Es sind eine Hülse 71, ein Spannkäfig 73 sowie ein Druckelement 78 vorgesehen. Die Hülse 71 ist hohlzylinderartig ausgeführt mit einer von einem hinteren zu einem vorderen Ende hin durchlaufenden Öffnung 72. Sie weist in ihrem hinteren Bereich ein Innengewinde und in ihrem vorderen Bereich einen kugelkalottenförmig ausgebildeten Passsitz für den Spannkäfig 73 auf. Der Spannkäfig 73 ist ähnlich zu der Fixierhülse 63 ausgeführt und weist vorzugsweise dieselben Abmessungen auf. Der Kerndurchmesser des Innengewindes ist so gewählt, dass der Spannkäfig 73 durch das Innengewinde hindurch an seinen Passsitz geschoben werden kann. Die Hülse 71 weist weiter zwei diametral gegenüberliegende Längsschlitze 74 auf. Sie erstrecken sich vom hinteren Ende der Hülse 71 über den gesamten Bereich des Innengewindes bis in den Bereich des Passsitzes der Durchgangsöffnung 72. Der Spannkäfig 73 ist genauso schwenkbar in seinem Passsitz gelagert wie die Fixierhülse 63 in dem Aufnahmesitz 61 des Fusionsmoduls 6.

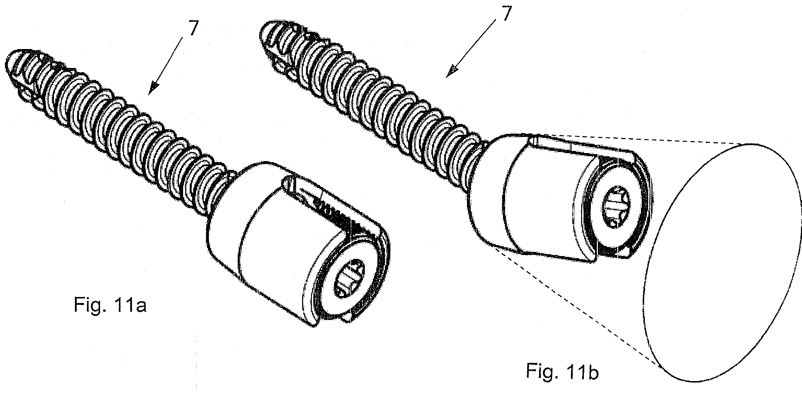

Im Spannkäfig 73 ist die Pedikelschraube 75 eingesteckt, und zwar derart, dass sie mit ihrem Kopf in den Spannkäfig aufgenommen ist. Der in den Schlitz 74 gesteckte Tragstab 70 drückt dabei auf den Kopf der Pedikelschraube 75. Indem die Madenschraube 78 in das Innengewinde der Durchgangsöffnung 72 soweit eingeschraubt wird, bis die Madenschraube 78 an dem Tragstab 70 anschlägt, kann durch weiteres Einschrauben der Tragstab 70 gegen den Kopf der Schraube 75, und dieser wiederum über den Spannkäfig 73 so verspannt werden, dass die Pedikelschraube 75 in ihrer axialen Orientierung zur Hülse 71 festgeklemmt ist. Damit ist eine polyaxiale Lagerung der Pedikelschraube erreicht, und zwar in einem Winkelbereich von ± 15° um die Mittelachse der Hülse 71 (siehe Fig. 11b). Die schwenkbare Ausführung ist nicht zwingend, es kann auch vorgesehen sein, dass die Pedikelschraube 75 sich in linearer Fortsetzung der Achse der Hülse 71 befindet, wie in Fig. 11a dargestellt. Durch Einschrauben der Pedikelschraube 75 in den Pedikel 91 des Wirbels 9 und anschließendem Verspannen mittels der Madenschraube 78 kann damit eine zusätzliche Befestigung erreicht werden.

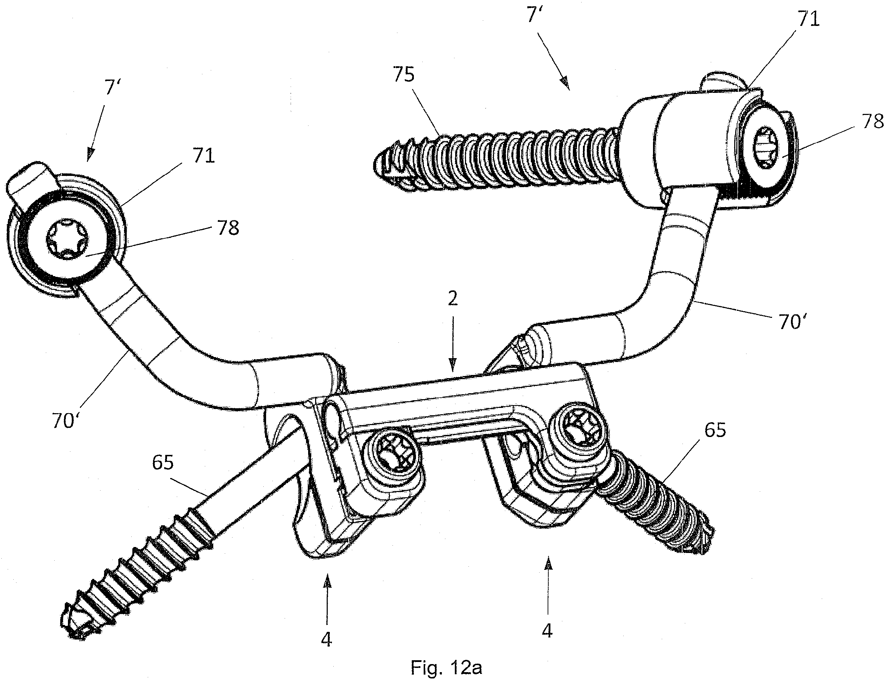

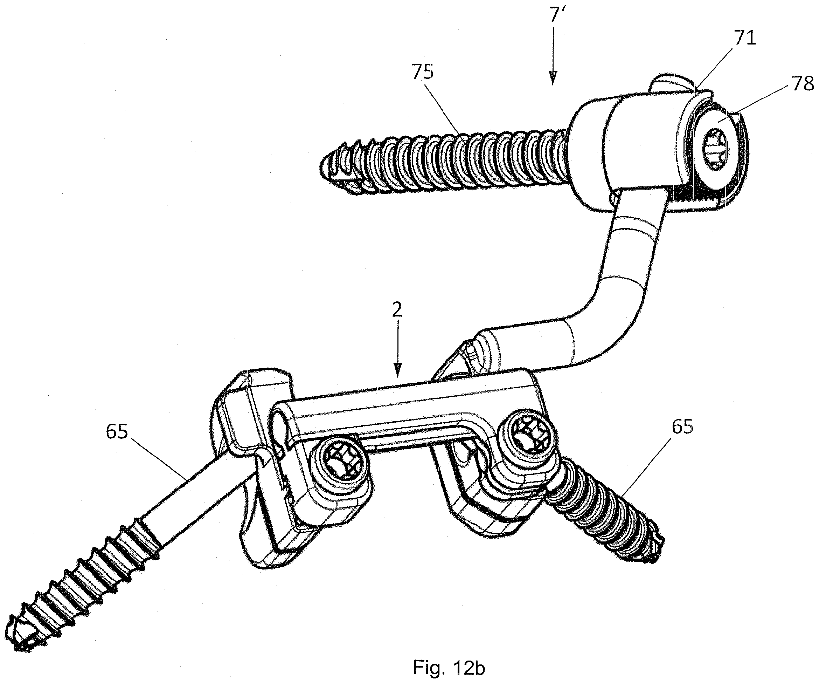

Bei einer Variante des vierten Ausführungsbeispiels ist der Tragstab bogenförmig ausgeführt und lädt weit nach lateral aus (siehe Fig. 12a). Damit kann ebenfalls eine Pedikelschraubenfixierung erreicht werden, die jedoch im Gegensatz zu der in Fig. 11a, b dargestellten Variante eine laterale Anordnung der Pedikelschraube 75 vorsieht. Die in Fig. 11a, b dargestellte Variante bewirkt hingegen eine mediale Anordnung der Pedikelschrauben. Die laterale Anordnung kann den Vorteil für sich buchen, eine Abstützung auf einer breiteren Basis zu schaffen, hat jedoch den mitunter beträchtlichen Nachteil, dass sie wegen ihrer ausladenden Konstruktion zu einer erhöhten Irritation des umliegenden Gewebes führt. Es ist nicht zwingend erforderlich, dass die laterale Abstützung auf beiden Seiten erfolgt. Es kann bei weiteren Varianten auch vorgesehen sein, dass der Tragstab einstückig mit dem Backenelement ausgeführt ist und/oder dass die Pedikelschraube lediglich einseitig vorgesehen ist (siehe Fig. 12b).

Bei dem in Fig. 13 dargestellten Ausführungsbeispiel handelt es sich um eine Variante des dritten Ausführungsbeispiels, wie in Fig. 8 dargestellt. Es weist im Unterschied zu der in Fig. 8 dargestellten Ausführungsform kürzere Backenelemente 4' auf und ist ferner mit einer Pedikelstütze 7' zur zusätzlichen Befestigung versehen. Die Pedikelstütze 7' ist funktionsgleich und im Wesentlichen ähnlich wie in Fig. 10 dargestellt aufgebaut, wobei gleichartige Elemente mit denselben Bezugsziffern versehen sind. Ein wesentlicher Unterschied liegt darin, dass der Tragstab 70' an dem Backenelement 4' selbst und nicht an dem Tragkörper 1 gehaltert ist. Dazu ist das Backenelement mit einer zusätzlichen Aufnahmebohrung versehen, in die eine gesonderte Klemmschraube 77 mit einem konusförmigen Kopf 77' eingesetzt ist. Der Tragstab 70' ist zur Befestigung mit einer augenförmigen Aufnahme 76 versehen. In diese ist ein kalottenförmiger geschlitzter Lagerkörper 63' eingesetzt, der wie der Lagerkörper 63 ausgeführt und mit einer Durchgangsöffnung versehen ist. Durch die Durchgangsöffnung ist die Klemmschraube 77 gesteckt und bildet so ein Schwenklager, welches unter der Wirkung der in den geschlitzten Lagerkörper 63' gesteckten Klemmschraube 77 arretiert werden kann. Zur wirksamen Arretierung ist der Lagerkörper 63' mit einer nach innen weisenden Radialleiste 69 versehen, an der ein kurzes Gegengewinde (zwei Umdrehungen) für die Klemmschraube 77 angeordnet ist. Die Radialleiste 69 teilt den Innenraum des Lagerkörpers 63' in einen kürzeren zylindrischen Teil 69'' und einen längeren konischen Teil 69'. Der Konuswinkel des konischen Teils 69' ist komplementär zu dem des Kopfs 77' der Klemmschraube 77. Damit wird zweierlei bewirkt. Zum einen fasst das Gewinde der Klemmschraube 77 in das Gegengewinde des Lagerkörpers 63' und zieht beim Einschrauben beide Komponenten eng zusammen, und zum anderen erfolgt durch die Konizität ein Aufspreizen des Lagerkörpers 63', was zu einer nochmaligen Steigerung der Fixierung des Schwenkwinkelsposition des Tragstabs 70' führt. Zur Erzielung einer eindeutigen Positionierung innerhalb des Auges 76 ist der Lagerkörper 63' an einem Rand der Durchgangsöffnung mit einem umlaufenden Ringkragen 64 versehen. Er ist so bemessen, dass er in die Aufnahmebohrung an dem Backenelement 4' fasst und so eine Normallage für den kalottenförmigen Lagerkörper 63' definiert. Damit sind die möglichen Schwenkwinkel für die Pedikelstütze 7' definiert.

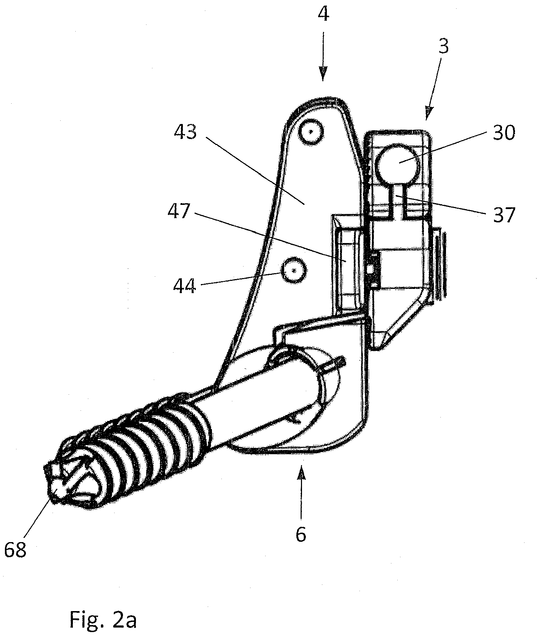

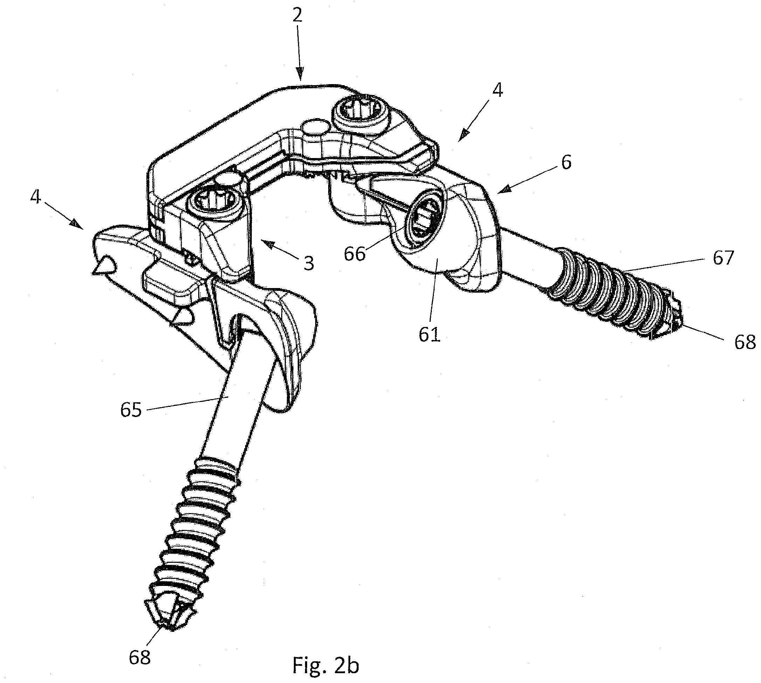

Nachfolgend wird das zur Implantation vorgesehene Instrumentarium beschrieben. Es umfasst einen Führungsdraht 80, einen Führungsschaft 81, ein Gewebeschutzrohr 82, einen kanülierten Schraubendreher 83, einen weiteren Schraubendreher 84, eine Pinzette 85 sowie eine Spreizzange 87. Die Implantation erfolgt bei einem Fusionsimplantat gemäß dem ersten Ausführungsbeispiel in der folgenden Weise. Zuerst wird mittels eines geeigneten Resektionsinstruments (nicht dargestellt), wie es an sich aus dem Stand der Technik bekannt ist, eine für das erste Ausführungsbeispiel vollständige Resektion des rückwärtigen Teils der Lamina mit dem spinalen Vorsprung. Es werden damit zwei laterale Laminaresektionsflächen 94 gebildet. Zwischen ihnen entsteht ein Freiraum, der Zugang zu dem Kanal gibt. Es kann nun auf an sich bekannte Weise eine Dekompression durchgeführt werden. Ist dies erfolgt, wird das Implantat gemäß dem ersten Ausführungsbeispiel eingesetzt. Es wird dazu an seinen Ort gesetzt mittels der Setzpinzette 85. Dies geschieht in der Weise, in dem Aufnahmespitzen 86 der Setzpinzette 85 in Aufnahmeöffnungen 26 an der Oberseite des Tragmoduls 1 eingesteckt werden. Die Aufnahmeöffnungen 26 können wie in Fig. 1 dargestellt, gesonderte Öffnungen sein, oder es kann sich wie in Fig. 2 dargestellt, um eine Kombination mit ohnehin vorhandenen Öffnungen zur Werkzeugaufnahme am Schraubenkopf 51 der Ausrichteinrichtung 5 handeln. Die Aufnahme des Implantats an der Setzpinzette 85 erfolgt über Reibschluss.

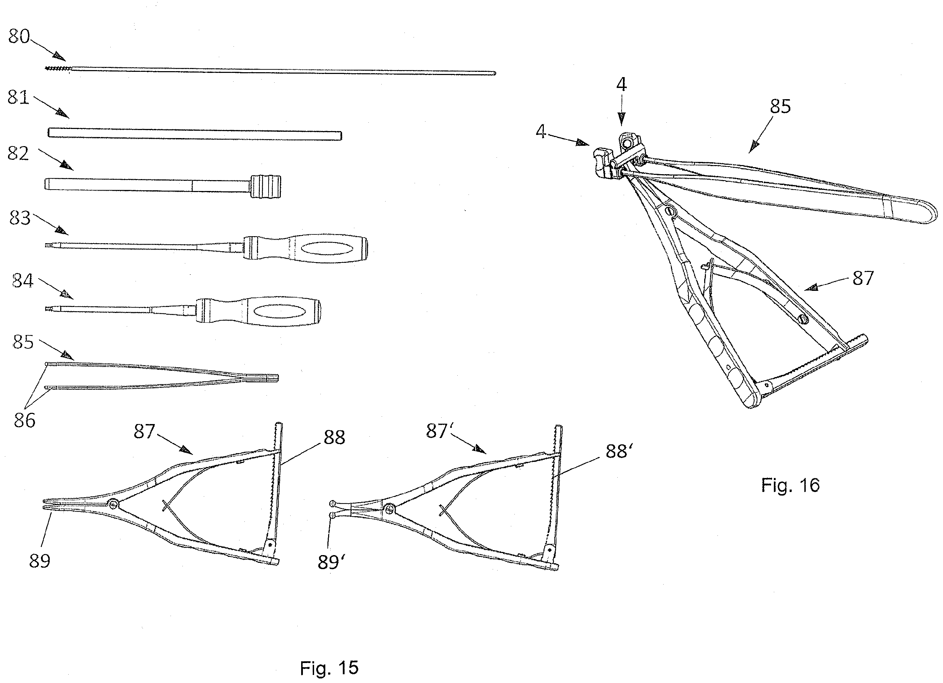

Das Implantat wird an seinen vorgesehenen Implantationsort im Freiraum zwischen den Lamina-Resektionsflächen 94 geführt und mittels einer Spreizzange 87 gespreizt. Dadurch entfernt sich der Schlitten 3 von dem Kulissenkörper 2 derart, dass die Backenelemente 4 sich soweit auseinander bewegen, bis sie mit ihren Außenflächen 43 an den Lamina-Resektionsflächen 94 anliegen. Dabei wird die Spreizzange 87 gesetzt, bevor die Setzpinzette 85 abgenommen wird. Dies geschieht auf die in Fig. 16 dargestellte Weise, nämlich indem die Spreizzange 87 von unten zugeführt wird. Die Spreizzange 87 weist an ihrem vorderen Ende Greifelemente 89 auf, die an eine entsprechende Gegenfläche an der Innenseite der Backenelemente 4 eingreifen. Die Greifelemente 89 können insbesondere als Greifkugeln 89' ausgeführt sein, welche in Greifmulden 29 an den Innenseiten der Backenelemente 4 formschlüssig eingreifen. Ist die Spreizzange 87' wie in Fig. 12 dargestellt in Eingriff mit den Backenelementen 4 gebracht, so kann mittels der Ratsche 88' die Spreizstellung der Spreizzange fixiert werden. Die Setzpinzette 85 kann nun abgenommen werden. Das Implantat ist unter der Wirkung der Spreizzange 87' an seinem Ort gehaltert. Die Spreizzange 87' ist so nach cephalad abgewinkelt geformt, dass auch im angesetzten Zustand Zugang zu dem Fusionsmodul 6, und insbesondere die darin einzusetzende transfacettale Schraube 65 besteht. Damit ist eine Primärpositionierung erreicht.

Zum positionsgenauen Setzen der transfacettalen Schraube 65 wird der Führungsschaft 81 in der richtigen Orientierung in die Öffnung 62 des Fusionsmoduls 6 eingesetzt. Dies kann unter Röntgenkontrolle folgen. Ist der Führungsschaft 81 richtig positioniert, wird der Führungsdraht 80 durch den Schaft eingesetzt und durch die Facetten 96, 97 bewegt. Hat der Führungsdraht 80 seine Position erreicht, wird der Führungsschaft 81 durch ein Gewebeschutzrohr 82 ersetzt. Die kanülierte Facettenschraube 65 wird mit ihrer Hohlbohrung 68 auf dem Führungsdraht 80 aufgefädelt und mithilfe des gleichfalls kanülierten Schraubendrehers 83 durch das Gewebeschutzrohr 82 zu dem Fusionsmodul 6 geführt und eingeschraubt. Dank der Kanülierung ist ein Festziehen der Schraube mittels des Schraubendrehers 83 ermöglicht, wobei die Positionierung durch den Führungsdraht 80 gewährleistet ist. Ist die Schraube 65 festgedreht, kann der Schraubendreher 83 entnommen und der Führungsdraht 80 mit dem Gewebeschutzrohr 82 entnommen werden. Derselbe Vorgang wird für die kontralaterale Facettenschraube 65 auf der anderen Seiten durchgeführt. Sind beide Facettenschrauben 65 angezogen, kann die Ausrichteinrichtung 5 durch Festziehen der Klemmschrauben 50 mit dem Schraubendreher 84 betätigt werden, und somit die Spreizposition des Tragmoduls 1 fixiert werden. Die Spreizzange 87' kann nunmehr abgenommen werden. Das Implantat ist an seinem Ort fixiert.

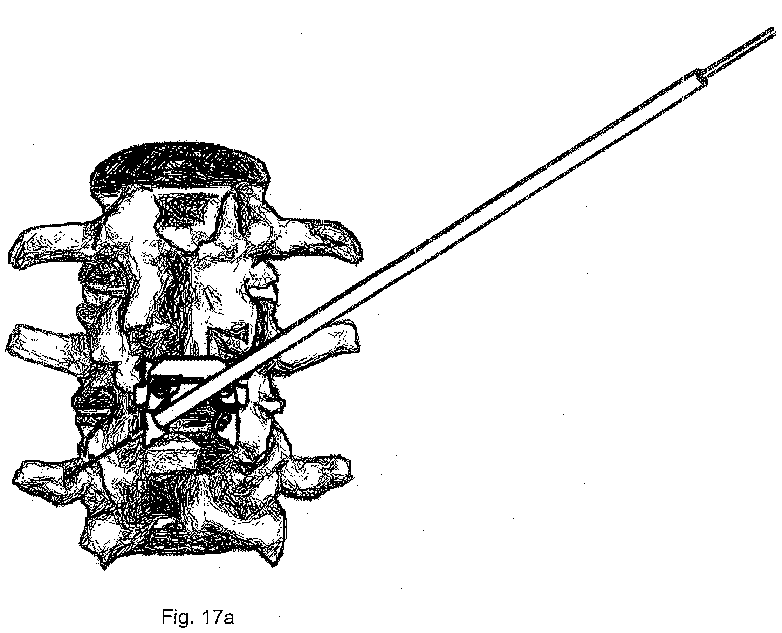

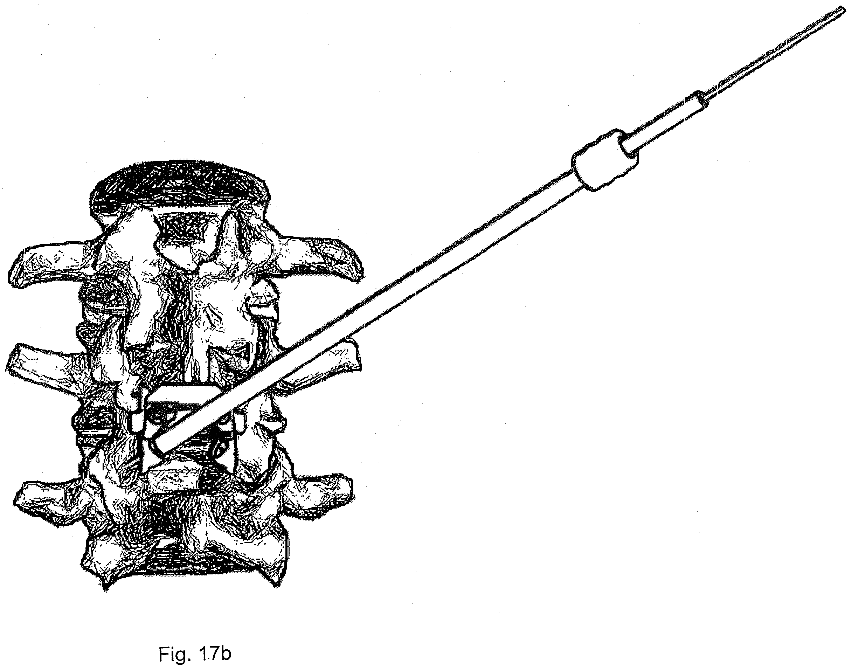

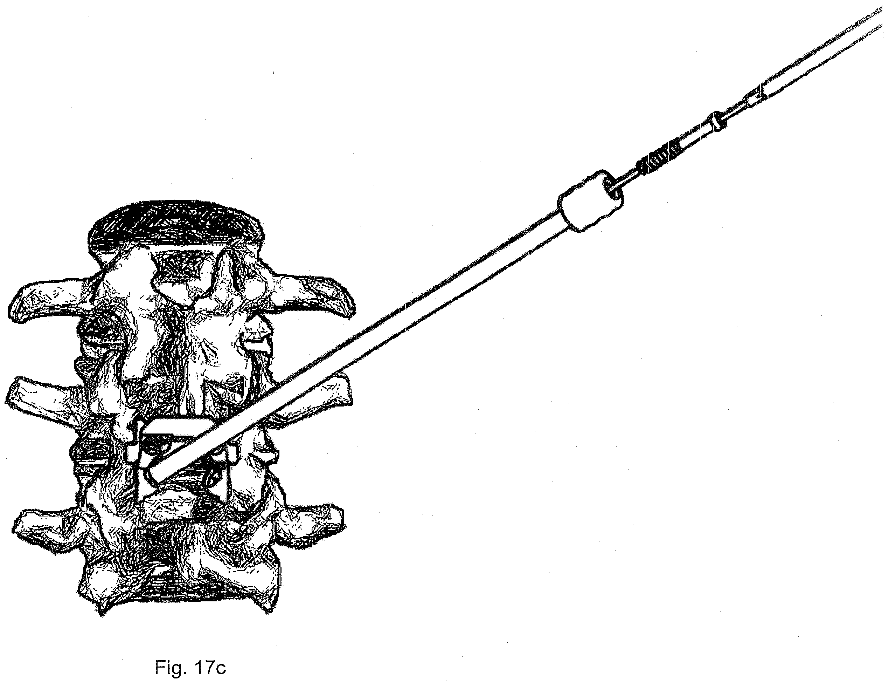

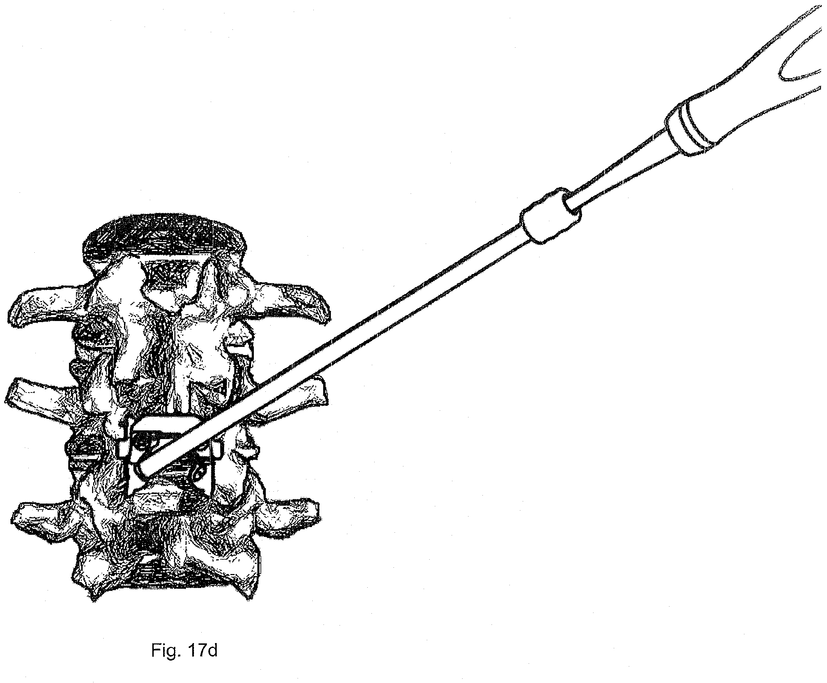

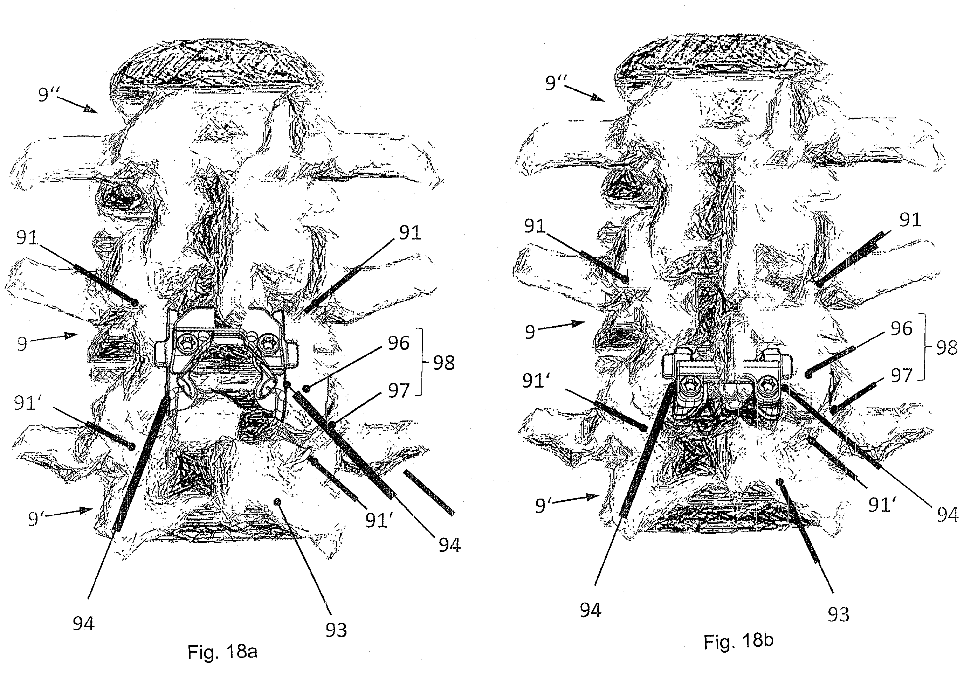

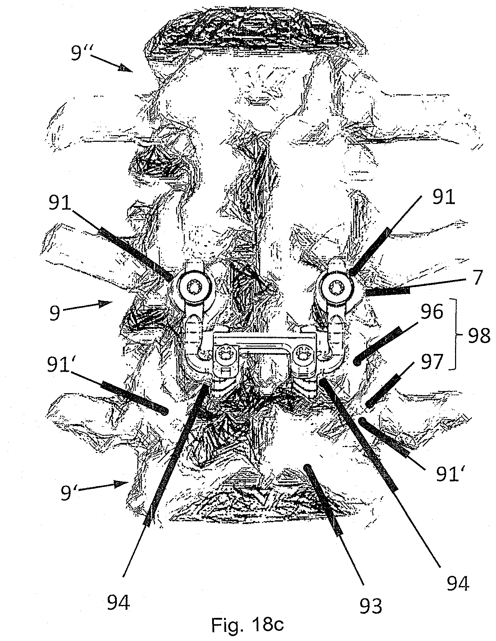

Die somit erreichte Einbauposition ist in Fig. 18a für das Implantat gemäß dem ersten Ausführungsbeispiel dargestellt. Man erkennt, dass das Implantat Lamina des Wirbels 9 vollständig ersetzt. In Fig. 17b ist das zweite Ausführungsbeispiel im implantierten Zustand dargestellt. Dieses Implantat hat eine geringere Bauhöhe und ermöglicht einen teilweisen Erhalt der Lamina, nämlich in ihrem oberen Bereich mit dem spinalen Vorsprung. Diese schonende Variante ist in Fig. 18b dargestellt. Die Implantation des vierten Ausführungsbeispiels mit den Pedikelschrauben gemäß Fig. 10 ist in Fig. 18c dargestellt.

## Patentansprüche

1. Fusionsimplantat für ein Facettengelenk, umfassend transfacettale Befestigungsmittel (65), ein Tragmodul (1) und Backenelemente (4, 4'), wobei die Backenelemente (4, 4') an dem Tragmodul (1) angeordnet sind und je einen Halter (61, 62) für die transfacettalen Befestigungsmittel (65) aufweisen,
**dadurch gekennzeichnet, dass**
das Tragmodul (1) ein Expansionselement (2, 3) umfasst, wobei die Backenelemente (4, 4') an voneinander wegweisenden Außenseiten Anlageflächen (43) für die Lamina (93) aufweisen und an einer Führung (28, 31) des Expansionselements (2, 3) so angeordnet sind, dass sie auseinander spreizbar sind, so dass ein Abstand der Backenelemente (4, 4') mittels des Expansionselements (2, 3) veränderlich ist.

2. Fusionsimplantat nach Anspruch 1,
**dadurch gekennzeichnet, dass**
ein transfacettales Befestigungsmittel und je einer der Halter für das transfacettale Befestigungsmittel (65) jeweils ein Fusionsmodul (6) bilden.

3. Fusionsimplantat nach Anspruch 2,
**dadurch gekennzeichnet, dass**
ein Hauptlager (60) des jeweiligen Halters für das transfacettale Befestigungsmittel (65) als ein polyaxiales Lager ausgeführt ist.

4. Fusionsimplantat nach Anspruch 3,
**dadurch gekennzeichnet, dass**
das Hauptlager (60) eine in einem kugelkalottenförmigen Aufnahmesitz (61) schwenkbar gehalterte Fixierhülse (63) umfasst.

5. Fusionsimplantat nach einem der Ansprüche 2 bis 4,
**dadurch gekennzeichnet, dass**
das transfacettale Befestigungsmittel als eine transfacettale Schraube (65) ausgeführt ist.

6. Fusionsimplantat nach Anspruch 5,
**dadurch gekennzeichnet, dass**
die transfacettale Schraube (67) ein Gewinde (67) aufweist, das als ein Gegenlager an einer unteren Facette fungiert.

7. Fusionsimplantat nach einem der vorangehenden Ansprüche, **dadurch gekennzeichnet, dass**
die Backenelemente (4, 4') eine im cephaladen Bereich schmalere Anlagefläche (43) als im caudalen Bereich aufweisen.

8. Fusionsimplantat nach einem der vorangehenden Ansprüche, **dadurch gekennzeichnet, dass**
der Halter für das transfacettale Befestigungsmittel (65) im caudalen Bereich des jeweiligen Backenelements (4, 4') angeordnet ist.

9. Fusionsimplantat nach einem der vorangehenden Ansprüche, **dadurch gekennzeichnet, dass**
das Tragmodul (1) in cephalad-caudaler Richtung eine Abmessung aufweist, die weniger als die Hälfte der Erstreckung der Anlagefläche (43) in dieser Richtung beträgt.

10. Fusionsimplantat nach Anspruch 9,
**dadurch gekennzeichnet, dass**
der Halter für das transfacettale Befestigungsmittel (65) im mittleren Bereich des jeweiligen Backenelements (4, 4') angeordnet ist.

11. Fusionsimplantat nach einem der Ansprüche 8 bis 10,
**dadurch gekennzeichnet, dass**
der Halter für das transfacettale Befestigungsmittel (65) an dem jeweiligen Backenelement (4, 4') in dessen tragmodulseitigen Bereich angeordnet ist.

12. Fusionsimplantat nach einem der Ansprüche 5 bis 11,
**dadurch gekennzeichnet, dass**
ein koaxialer Zugangsweg zu der transfacettalen Schraube (65) frei vom Tragmodul (1) ist.

13. Fusionsimplantat nach einem der vorangehenden Ansprüche, **dadurch gekennzeichnet, dass**
eine Pedikelstütze (7) an dem Tragmodul (1) angeordnet ist.

14. Fusionsimplantat nach Anspruch 13,
**dadurch gekennzeichnet, dass**
die Pedikelstütze (7) mittels einer Ausrichteinrichtung (5) gehaltert ist.

15. Fusionsimplantat nach einem der Ansprüche 1 bis 12,
**dadurch gekennzeichnet, dass**
eine Pedikelstütze (7') an dem Backenelement (4') angeordnet ist.

16. Fusionsimplantat nach Anspruch 15,
**dadurch gekennzeichnet, dass**
die Pedikelstütze (7') über ein Schwenklager an dem Backenelement (4') angeordnet ist.

17. Fusionsimplantat nach Anspruch 15 oder 16,
**dadurch gekennzeichnet, dass**
das Schwenklager einen kalottenförmigen Lagerkörper (63') mit einer Durchgangsöffnung aufweist, der vorzugsweise geschlitzt ist.

18. Fusionsimplantat nach Anspruch 17,
**dadurch gekennzeichnet, dass**
der kalottenförmige Lagerkörper (63') in seiner Durchgangsöffnung mit einer nach innen weisenden Radialleiste (69) mit einem Gegengewinde versehen ist und/oder am Rand der Durchgangsöffnung einen umlaufenden Ringkragen (64) aufweist.

19. Fusionsimplantat nach einem der Ansprüche 13 bis 18,
**dadurch gekennzeichnet, dass**
die Pedikelstütze (7, 7') ein Lager für Pedikelschrauben (75) aufweist.

20. Fusionsimplantat nach Anspruch 19, umfassend Pedikelschrauben (75) die parallele Achsen aufweisen die Spreizbarkeit quer zu einer Richtung der der liegen. Backenelemente (4, 4')

21. Fusionsimplantat nach einem der Ansprüche 13 bis 20,
**dadurch gekennzeichnet, dass**
die lateralen Abmessungen der Pedikelstütze (7, 7') maximal das 1,5-fache der lateralen Abmessungen des Tragmoduls (1) sind.

22. Fusionsimplantat nach einem der Ansprüche 19 bis 21,
**dadurch gekennzeichnet, dass**
des Lagers für die Pedikelstütze (7, 7') nach lateral ausgreifend ist und eine Achse Pedikelschrauben (75) zur Mitte des Tragmoduls (1) hin konvergierend ausgerichtet ist.

23. Fusionsimplantat nach einem der Ansprüche 19 und 21,
**dadurch gekennzeichnet, dass**
die Pedikelstütze (7, 7') eine polyaxiale Lagerung für die Pedikelschraube (75) aufweist.

24. Fusionsimplantat nach einem der vorangehenden Ansprüche, **dadurch gekennzeichnet, dass**
mindestens eines der Backenelemente (4, 4') mit einer Ausrichteinrichtung (5) zur veränderbaren Orientierung des Backenelements (4, 4') zum Tragmodul (1) versehen ist.

25. Fusionsimplantat nach Anspruch 24,
**dadurch gekennzeichnet, dass**
eine Klemmschraube (50) vorgesehen ist, die als Lager für die Ausrichteinrichtung (5) vorgesehen ist.

26. Fusionsimplantat nach Anspruch 24 oder 25,
**dadurch gekennzeichnet, dass**
die Ausrichteinrichtung (5) eine Verdrehsicherung (45, 55) aufweist, die zum Begrenzen eines Verdrehwinkels auf einen vorgegebenen Wert ausgebildet ist.

27. Fusionsimplantat nach einem der vorangehenden Ansprüche, **dadurch gekennzeichnet, dass**
ein zweites der Backenelemente (4) mit einer Ausrichteinrichtung (5) versehen ist.

28. Fusionsimplantat nach einem der vorangegangenen Ansprüche, **dadurch gekennzeichnet, dass**
das Expansionselement als Führung einen Stab (30) an einem Schlitten (3) und eine komplementär geformte Nut (28) an einem Kulissenkörper (2) umfasst.

29. Fusionsimplantat nach Anspruch 28,
**dadurch gekennzeichnet, dass**
der Stab (30) und die Nut (28) über eine in einen Schlitz formschlüssig eingreifende Leiste verdrehgesichert sind.

30. Fusionsimplantat nach Anspruch 28 oder 29,
**dadurch gekennzeichnet, dass**
am freien Ende des Stabs (30) eine Verdickung (39) ausgebildet ist.

31. Fusionsimplantat nach einem der vorangehenden Ansprüche, **dadurch gekennzeichnet, dass**
die Anlagefläche (43) spitze Hervorstehungen (46) und/oder eine knochenwachstumsfördernde Beschichtung aufweist.

32. Satz umfassend ein Fusionsimplantat nach einem der Ansprüche 1 bis 31 und ein Instrumentarium,
**dadurch gekennzeichnet, dass**
das Instrumentarium umfasst:
- einen langgestreckten Führungsdraht (80),
- einen Führungsschaft (81), durch welchen der Führungsdraht (80) steckbar ist, mit einem zur Aufnahme am Hauptlager (60) des Fusionsmoduls (6) ausgebildetem Ende, und
- einen kanülierten Schraubendreher (83).

33. Satz nach Anspruch 32,
**dadurch gekennzeichnet, dass**
das Instrumentarium weiter umfasst:
- eine Spreizzange (87, 87'), die vorzugsweise in ihrer Arbeitsstellung nach cephalad abgewinkelt ist und einen freien Zugang für den Führungsschaft gibt, und
- vorzugsweise eine gesonderte Setzpinzette vorgesehen ist, die vorzugsweise über einen reibschlüssige Verbindung mit dem Implantat verbunden ist.

## Claims

1. Fusion implant for a facet joint, comprising transfacetal fastening means (65), a support module (1) and jaw elements (4, 4'), wherein the jaw elements (4, 4') are arranged on the support module (1) and each has a holder (61, 62) for the transfacetal fastening means (65), **characterized in that** the support module (1) comprises an expansion element (2, 3), wherein the jaw elements (4, 4') have contact surfaces (43) for the lamina (93) on outer sides facing away from one another and are arranged on a guide (28, 31) of the expansion element (2, 3) such that they can be spread out from one another, such that a distance between the jaw elements (4, 4') is variable by means of the expansion element (2, 3).

2. Fusion implant according to Claim 1, **characterized in that** a transfacetal fastening means and each of the holders for the transfacetal fastening means (65) form in each case a fusion module (6).

3. Fusion implant according to Claim 2, **characterized in that** a main bearing (60) of the respective holder for the transfacetal fastening means (65) is designed as a polyaxial bearing.

4. Fusion implant according to Claim 3, **characterized in that** the main bearing (60) comprises a fixation sleeve (63) held pivotably in a spherical cap-shaped receiving seat (61).

5. Fusion implant according to one of Claims 2 to 4, **characterized in that** the transfacetal fastening means is designed as a transfacetal screw (65).

6. Fusion implant according to Claim 5, **characterized in that** the transfacetal screw (67) has a thread (67) which functions as a counterbearing on a lower facet.

7. Fusion implant according to one of the preceding claims, **characterized in that** the jaw elements (4, 4') have a narrower contact surface (43) in the cephalad area than in the caudal area.

8. Fusion implant according to one of the preceding claims, **characterized in that** the holder for the transfacetal fastening means (65) is arranged in the caudal area of the respective jaw element (4, 4').

9. Fusion implant according to one of the preceding claims, **characterized in that** the support module (1) has a dimension in a cephalad-caudal direction, which amounts to less than half of the extent of the contact surface (43) in said direction.

10. Fusion implant according to Claim 9, **characterized in that** the holder for the transfacetal fastening means (65) is arranged in the central area of the respective jaw element (4, 4').

11. Fusion implant according to one of Claims 8 to 10, **characterized in that** the holder for the transfacetal fastening means (65) is arranged on the respective jaw element (4, 4') in its support module side area.

12. Fusion implant according to one of Claims 5 to 11, **characterized in that** a coaxial access pathway to the transfacetal screw (65) is free of the support module (1) .

13. Fusion implant according to one of the preceding claims, **characterized in that** a pedicle support (7) is arranged on the support module (1).

14. Fusion implant according to Claim 13, **characterized in that** the pedicle support (7) is held by means of the alignment device (5).

15. Fusion implant according to one of Claims 1 to 12, **characterized in that** a pedicle support (7') is arranged on the jaw element (4').

16. Fusion implant according to Claim 15, **characterized in that** the pedicle support (7') is arranged on the jaw element (4') via a pivot bearing.

17. Fusion implant according to Claim 15 or 16, **characterized in that** the pivot bearing has a dome-shaped bearing body (63') with a pass-through opening, which preferably is slotted.

18. Fusion implant according to Claim 17, **characterized in that** the dome-shaped bearing body (63') in its pass-through opening is provided with a radial member (69) pointing inward and having a mating thread and/or has a circular ring collar (64) on the edge of the pass-through opening.

19. Fusion implant according to one of Claims 13 to 18, **characterized in that** the pedicle support (7, 7') has a bearing for pedicle screws (75).

20. Fusion implant according to Claim 19, comprising pedicle screws (75) which have parallel axes lying transversely with respect to a direction of the spreadability of the jaw elements (4, 4').

21. Fusion implant according to one of Claims 13 to 20, **characterized in that** the lateral dimensions of the pedicle support (7, 7') amount to a maximum of 1.5 times the lateral dimensions of the support module (1).

22. Fusion implant according to one of Claims 13 to 21, **characterized in that** the pedicle support (7, 7') protrudes laterally, and an axis of the bearing for pedicle screws (75) is aligned and converging toward the center of the support module (1).

23. Fusion implant according to one of Claims 19 and 21, **characterized in that** the pedicle support (7, 7') has a polyaxial support for the pedicle screw (75).

24. Fusion implant according to one of the preceding claims, **characterized in that** at least one of the jaw elements (4, 4') is provided with an alignment device (5) for variable orientation of the jaw element (4, 4') in relation to the support module (1).

25. Fusion implant according to Claim 24, **characterized in that** a locking screw (50) is provided as a bearing for the alignment device (5).

26. Fusion implant according to Claim 24 or 25, **characterized in that** the alignment device (5) has a twist-proof device (45, 55), which is designed for limiting an angle of twist to a predetermined level.

27. Fusion implant according to one of the preceding claims, **characterized in that** a second one of the jaw elements (4) is provided with an alignment device (5).

28. Fusion implant according to one of the preceding claims, **characterized in that** the expansion element comprises a rod (30) on a slide (3) and a groove (28) having a complementary shape on a rail body (2) as guide.

29. Fusion implant according to Claim 28, **characterized in that** the rod (30) and the groove (28) are secured to prevent twisting by means of a strip which engages in a slot in a form-fitting manner.

30. Fusion implant according to Claim 28 or 29, **characterized in that** a thickened part (39) is arranged on the free end of the rod (30).

31. Fusion implant according to one of the preceding claims, **characterized in that** the contact surface (43) has pointed protrusions (46) and/or a coating which promotes bone growth.

32. Set comprising a fusion implant according to one of Claims 1 to 31 and an instrument set, **characterized in that** the instrument set comprises:
- an elongated guide wire (80),
- a guide shaft (81) through which the guide wire (80) can be inserted, having one end designed for being received on the main bearing (60) of the fusion module (6), and
- a cannulated screwdriver (83).

33. Set according to Claim 32, **characterized in that** the instrument set further comprises:
- spreading forceps (87, 87') which are preferably angled in a cephalad direction in their working position and provide free access for the guide wire, and
- preferably separate setting pincettes, preferably being connected to the implant via a friction-engaged connection.

## Revendications

1. Implant de fusion, destiné à une articulation à facettes, comprenant des moyens de fixation transfacettaires (65), un module de support (1) et des éléments de mâchoire (4, 4'), les éléments de mâchoire (4, 4') étant disposés au niveau du module de support (1) et comprenant chacun un support (61, 62) destiné aux moyens de fixation transfacettaires (65), **caractérisé en ce que**
le module de support (1) comprend un élément d'expansion (2, 3), les éléments de mâchoire (4, 4') comportant sur des côtés extérieurs, tournés à l'opposé les uns des autres, des surfaces d'appui (43) destinées à la lamina (93) et étant disposés au niveau d'un guide (28, 31) de l'élément d'expansion (2, 3) de façon à pouvoir être écartés les uns des autres de sorte qu'une distance entre les éléments de mâchoire (4, 4') est modifiable au moyen de l'élément d'expansion (2, 3).

2. Implant de fusion selon la revendication 1, **caractérisé en ce que**
un moyen de fixation transfacettaire et l'un correspondant des supports destinés aux moyens de fixation transfacettaires (65) forment à chaque fois un module de fusion (6).

3. Implant de fusion selon la revendication 2, **caractérisé en ce que**
un palier principal (60) du support respectif destiné au moyen de fixation transfacettaire (65) est conçu comme un palier polyaxial.

4. Implant de fusion selon la revendication 3, **caractérisé en ce que**
le palier principal (60) comprend un manchon de fixation (63) monté de manière pivotante dans un siège de réception (61) en forme de calotte sphérique.

5. Implant de fusion selon l'une des revendications 2 à 4,
**caractérisé en ce que**
le moyen de fixation transfacettaire est conçu comme une vis transfacettaire (65).

6. Implant de fusion selon la revendication 5, **caractérisé en ce que**
la vis transfacettaire (67) comporte un filetage (67) faisant office de palier-support sur une facette inférieure.

7. Implant de fusion selon l'une des revendications précédentes,
**caractérisé en ce que**
les éléments de mâchoire (4, 4') comportent une surface d'appui (43) plus étroite dans la région céphalique que dans la région caudale.

8. Implant de fusion selon l'une des revendications précédentes,
**caractérisé en ce que**
le support destiné au moyen de fixation transfacettaire (65) est disposé dans la région caudale de l'élément de mâchoire respectif (4, 4').

9. Implant de fusion selon l'une des revendications précédentes,
**caractérisé en ce que**
le module de support (1) a dans la direction céphalocaudale une dimension qui est inférieure à la moitié de l'étendue de la surface d'appui (43) dans cette direction.

10. Implant de fusion selon la revendication 9, **caractérisé en ce que**
le support destiné au moyen de fixation transfacettaire (65) est disposé dans la région centrale de l'élément de mâchoire respectif (4, 4').

11. Implant de fusion selon l'une des revendications 8 à 10,
**caractérisé en ce que**
le support destiné au moyen de fixation transfacettaire (65) est disposé au niveau de l'élément de mâchoire respectif (4, 4') dans sa région côté module de support.

12. Implant de fusion selon l'une des revendications 5 à 11,
**caractérisé en ce que**
un chemin d'accès coaxial à la vis transfacettaire (65) est dépourvu du module de support (1).

13. Implant de fusion selon l'une des revendications précédentes,
**caractérisé en ce que**
un support pédiculaire (7) est disposé au niveau du module de support (1).

14. Implant de fusion selon la revendication 13, **caractérisé en ce que**
le support pédiculaire (7) est maintenu au moyen d'un dispositif d'orientation (5).

15. Implant de fusion selon l'une des revendications 1 à 12,
**caractérisé en ce que**
un support pédiculaire (7') est disposé au niveau de l'élément de mâchoire (4').

16. Implant de fusion selon la revendication 15, **caractérisé en ce que**
le support pédiculaire (7') est disposé au niveau l'élément de mâchoire (4') par le biais d'un palier de pivotement.

17. Implant de fusion selon la revendication 15 ou 16, **caractérisé en ce que**
le palier de pivotement comporte un corps de palier (63') en forme de calotte pourvu d'une ouverture de passage, de préférence fendu.

18. Implant de fusion selon la revendication 17, **caractérisé en ce que**
le corps de palier (63') en forme de calotte est pourvu, dans son ouverture de passage, d'une baguette radiale (69) dirigée vers l'intérieur et munie d'un contre-filet et/ou comporte une collerette annulaire circonférentielle (64) au niveau du bord de l'ouverture de passage.

19. Implant de fusion selon l'une des revendications 13 à 18,
**caractérisé en ce que**
le support pédiculaire (7, 7') comporte un palier destiné à des vis pédiculaires (75).

20. Implant de fusion selon la revendication 19, comprenant des vis pédiculaires (75) ayant des axes parallèles placés transversalement à une direction d'écartement des éléments de mâchoire (4, 4').

21. Implant de fusion selon l'une des revendications 13 à 20,
**caractérisé en ce que**
les dimensions latérales du support pédiculaire (7, 7') sont au maximum égales à 1,5 fois les dimensions latérales du module de support (1).

22. Implant de fusion selon l'une des revendications 19 à 21,
**caractérisé en ce que**
le support pédiculaire (7, 7') s'étend latéralement et un axe du palier destiné à des vis pédiculaire (75) est orienté de façon à converger vers le centre du module de support (1).

23. Implant de fusion selon l'une des revendications 19 et 21,
**caractérisé en ce que**
le support pédiculaire (7, 7') présente un agencement de palier polyaxial pour la vis pédiculaire (75).

24. Implant de fusion selon l'une des revendications précédentes,
**caractérisé en ce que**
l'un au moins des éléments de mâchoire (4, 4') est muni d'un dispositif d'orientation (5) destiné à orienter de manière modifiable l'élément de mâchoire (4, 4') vers le module de support (1).

25. Implant de fusion selon la revendication 24, **caractérisé en ce que**
il est prévu une vis de serrage (50) faisant office de palier pour le dispositif d'orientation (5).

26. Implant de fusion selon la revendication 24 ou 25, **caractérisé en ce que**
le dispositif d'orientation (5) comporte un dispositif anti-rotation (45, 55) conçu pour limiter un angle de rotation à une valeur prescrite.

27. Implant de fusion selon l'une des revendications précédentes,
**caractérisé en ce que**
un deuxième élément de mâchoire (4) est muni d'un dispositif d'orientation (5).

28. Implant de fusion selon l'une des revendications précédentes,
**caractérisé en ce que**
l'élément d'expansion comporte comme moyen de guidage une barre (30) montée sur un chariot (3) et une gorge (28) de forme complémentaire ménagée au niveau d'un corps de coulisse (2) .

29. Implant de fusion selon la revendication 28, **caractérisé en ce que**
la barre (30) et la gorge (28) sont immobilisées en rotation au moyen d'une baguette s'engageant dans une fente par complémentarité de formes.

30. Implant de fusion selon la revendication 28 ou 29, **caractérisé en ce que**
un épaississement (39) est formé à l'extrémité libre de la barre (30).

31. Implant de fusion selon l'une des revendications précédentes, **caractérisé en ce que**
la surface d'appui (43) comporte des saillies pointues (46) et/ou un revêtement favorisant la croissance des os.

32. Ensemble comprenant un implant de fusion selon l'une des revendications 1 à 31 et un ensemble d'instruments,
**caractérisé en ce que**
l'ensemble d'instruments comprend :
- un fil de guidage allongé (80),
- une tige de guidage (81), à travers laquelle le fil de guidage (80) peut être passé, pourvue d'une extrémité conçue pour être reçue au niveau du palier principal (60) du module de fusion (6), et
- un tournevis canulé (83).

33. Ensemble selon la revendication 32,
**caractérisé en ce que**
l'ensemble d'instruments comprend en outre :
- une pince extensible (87, 87'), qui est de préférence coudée dans sa position de travail vers la région céphalique et permet un accès libre à la tige de guidage, et
- de préférence une pincette de réglage séparée, qui est reliée à l'implant de préférence par une liaison par friction.
